(19) Europäisches Patentamt
European Patent Office
Office européen des brevets

(11) Publication number: **0 487 502 A2**

# (12) EUROPEAN PATENT APPLICATION

(21) Application number: **92100224.2**

(22) Date of filing: **18.06.90**

(51) Int. Cl.⁵: **C07D 313/12**, C07D 223/20, C07C 229/00, C07C 211/32, A61K 31/335, A61K 31/395

This application was filed on 09 - 01 - 1992 as a divisional application to the application mentioned under INID code 60.

(30) Priority: **19.06.89 GB 8914040**
**19.06.89 GB 8914060**
**19.06.89 GB 8914061**
**19.06.89 GB 8914062**

(43) Date of publication of application:
**27.05.92 Bulletin 92/22**

(60) Publication number of the earlier application in accordance with Art.76 EPC: **0 409 406**

(84) Designated Contracting States:
**AT BE CH DE DK ES FR GB GR IT LI LU NL SE**

(71) Applicant: **THE WELLCOME FOUNDATION LIMITED**
**Unicorn House 160 Euston Road**
**London NW1 2BP(GB)**

(72) Inventor: **Lever, Oscar William**
**14 Fenimore Drive**
**Pittsford, New York 14534(US)**
Inventor: **Harfenist, Morton**
**Route 6, Box 405**
**Chapel Hill, North Carolina 27514(US)**
Inventor: **King, Ann Christie**
**4 Cedronella Drive**
**Chapel Hill, North Carolina 27514(US)**
Inventor: **Chao, Esther Yu-Hsuan**
**221, Trails End Court**
**Raleigh, North Carolina 27614(US)**

(74) Representative: **Rollins, Anthony John et al**
**Group Patents & Agreements The Wellcome Research Laboratories Langley Court**
**Beckenham Kent BR3 3BS(GB)**

(54) **Pharmaceutically active aryl-substituted amine derivatives.**

(57) Compounds of formula (IVa)

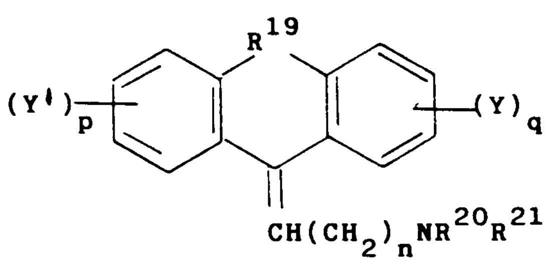

(IVa)

wherein:
$R^{19}$ is $-CH_2CH_2-$, $-CH_2O-$, $-OCH_2-$, $-NHCH_2-$, or $-CH_2NH-$;
Y is hydrogen or halogen attached at the 1, 2, 3 or 4 position;
Y' is hydrogen or halogen attached at the 7, 8, 9 or 10 position;
n is 0 to 3;

EP 0 487 502 A2

each of p and q is 1 to 4; and

$R^{20}$ and $R^{21}$ independently are hydrogen, or $C_{1-4}$ alkyl, or together with the nitrogen atom form a nitrogen-containing heterocyclic ring having four to six ring members, with the provisos that

a) when $R^{19}$ is $-CH_2CH_2-$, $-CH_2O-$ or $-OCH_2-$, p and q are each 1 and one of Y and Y' is halogen, the other is not hydrogen; and

b) when $R^{19}$ is $-CH_2CH_2-$ or $-CH_2O-$, p and q are both 1 and each of Y and Y' is hydrogen or halogen, then n is not 2;

or pharmaceutically acceptable salts thereof, process for their preparation and pharmaceutical formulations containing them.

The present invention relates to the use of certain aryl-substituted amine derivatives as adjuvant chemotherapy for neoplasias resistant to multiple drugs. The present invention also relates to the use of such compounds as agents for enhancing the therapeutic effect of multiple antitumor agents.

Complete cures of various tumors like leukemias, lymphomas and solid tumors by the use of chemotherapeutic agents are rare because of heterogeneous sensitivity of tumor cells to each antitumor agent. Cancer chemotherapy also fails because of intrinsic resistance of tumors to multiple drug therapies. In other cases, a tumor may become resistant to the antitumor agents used in a previous treatment. The therapeutic effects of these agents are then eliminated. An even graver problem is that recurrent cancers are resistant not only to the cancer suppressants used in previous treatments, but also manifest resistance to other antitumor agents, unrelated to the agent used previously either by chemical structure or by mechanism of action. These phenomenon are collectively referred to as multiple drug resistance (mdr) and contribute widely to cancer treatment failures in the clinic.

The major documented cause of multiple drug resistance is overexpression of a membrane glycoprotein (the multiple drug transporter) responsible for pumping structurally diverse antitumor drugs from cells. See D. Houseman et al., A Molecular Genetic Approach to the Problem of Drug Resistance in Chemotherapy, 504-517 (1987) (Academic Press, Inc.); R. Fine and B. Chabner, Multidrug Resistance, in Cancer Chemotherapy 8, 117-128 (H. Pinedo and B. Chabner eds. 1986).

Tumor cells expressing elevated levels of the multiple drug transporter accumulate far less antitumor agents intracellularly than tumor cells having low levels of this enzyme. The degree of resistance of certain tumor cells has been documented to correlate with both elevated expression of the drug transporter and reduced accumulation of antitumor drugs. See M. Gottesman and I. Pastan, J. Biol. Chem. 263, 12163 (1988); see also A. Fojo et al., Cancer Res. 45, 3002 (1985). This form of multiple drug cross-resistance involves agents derived from natural products, such as the vinca alkaloids, the anthracyclines, the epipodophyllotoxins, actinomycin D and plicamycin. See I. Pastan and M. Gottesman, New England J. Med. 1388, 1389 Table 1 (May 28, 1987).

Adenocarcinomas derived from adrenal, kidney, liver, small intestine, and colon tissue are notorious for exhibiting inherent cross-resistance to chemically unrelated chemotherapeutic agents. See M. Gottesman and I. Pastan, supra at 12165; see also A. Fojo et al., J. Clin. Oncol. 5, 1922 (1987). These tissues normally express higher levels of the multidrug transporter. Other tumors documented to express high levels of the multidrug transporter include pancreatic, carcinoid, chronic myelogenous leukemia in blast crisis, and non-small cell lung carcinoma. Tumors documented to be initially drug-sensitive but then to become drug resistant include neuroblastoma, pheochromocytoma, acute lymphocytic leukemia in adults, acute nonlymphocytic leukemia in adults, nodular poorly differentiated lymphoma, breast cancer and ovarian cancers. It is estimated by the National Cancer Institute that approximately half a million tumor samples a year will be drug resistant because of aberrant levels of expression of the multidrug transporter. See L. Goldstein et al., Expression of Multidrug Resistance Gene in Human Cancers, J. National Cancer Institute 81, 116 (1988).

Elevated levels of expression of the multidrug transporter in these tumors would lead to reduced intracellular levels of antitumor agents in the tumor and would cause suppression of chemotherapeutic efficacy. Tumors having elevated levels of the multidrug transporter would require therapeutic doses of cancer suppressants far in excess of tumors exhibiting lower levels of the multi drug transporter. Agents that inhibit the active efflux of antitumor agents by the drug transporter or agents that potentiate the efficacy of chemotherapeutic agents would enhance the activity of various antitumor agents on tumor cells. As a result of the present inventors' study, it has unexpectedly been found that when the compounds disclosed herein are used together with an antitumor agent, they can remarkably enhance the therapeutic effect of the antitumor agent, and that multiple drug resistance is resolved by increasing the susceptibility to actinomycin D.

A number of agents used clinically as calcium channel-blockers, calmodulin inhibitors and antiarrhythmic agents promote the activity of antitumor agents against resistant tumor cells, see Tsuruo et al., Cancer Res. 44, 4303 (1984); 43, 2267 (1983). Verapamil, caroverine, clomipramine, trifluoperazine, prenylamine, diltiazem, nicardipine, and quinidine enhance the activity of antitumor agents against resistant sublines of murine leukemia cells. Most agents potentiating the activity of antitumor agents are calcium antagonists, and the serious cardiotoxicities that arise during treatment have limited their clinical usefulness. While the inventors do not wish to be bound by any theory of operation for the present invention, it is noted that the potentiating agents disclosed herein are not known to have calcium antagonism. Nevertheless, the present inventors have now found that these agents do elevate the intracellular concentration of antineoplastic drugs in tumor cells overexpressing the multiple drug transporter. Sensitization of drug resistant tumors and elevation of intracellular antitumor drug concentrations probably occur by a mechanism different from calcium antagonism.

In a first aspect the present invention provides, as an agent for enhancing the therapeutic effect of an antineoplastic agent, a compound of formula (I) below or a pharmaceutically acceptable salt thereof:

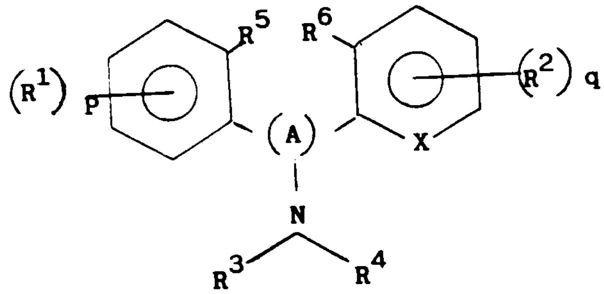

(I)

wherein A represents a group selected from:

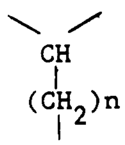

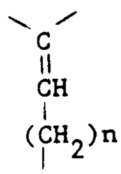

or

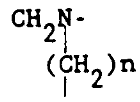

| | |
|---|---|
| X | represents -CH- or -N-; |
| $R^1$ | represents a hydrogen or halogen atom, a $C_{1-4}$ alkoxy group, a $C_{1-4}$ alkyl group, or a $C_{1-4}$ alkyl group substituted by 1 to 3 halogen atoms; |
| $R^2$ | represents a hydrogen or halogen atom, a $C_{1-4}$ alkoxy group, benzyloxy, a group $-R^7COOR^8$ (wherein $R^7$ represents a single bond or a $C_{1-7}$ bivalent aliphatic hydrocarbon group and $R^8$ represents a hydrogen atom or a $C_{1-4}$ alkyl group) or a group $R^7CONR^{8a}R^{8b}$ (wherein $R^{8a}$ and $R^{8b}$ each represent a hydrogen atom or a $C_{1-4}$ alkyl group or together with the nitrogen atom to which they are attached form a 4-6 membered saturated heterocyclic ring); |
| $R^3$ and $R^4$, | which may be the same or different each represent a hydrogen atom or a $C_{1-4}$ alkyl group, or |
| $R^3$ and $R^4$ | together with the nitrogen atom to which they are attached form a 4-6 membered saturated heterocyclic ring, which may optionally contain an oxygen atom or a further nitrogen atom, which may itself be substituted by a $C_{1-4}$ alkyl group; |
| $R^5$ and $R^6$ | each represent a hydrogen atom or together form a linking group between the aromatic rings, said group being selected from $-CH_2CH_2-$, $-CH_2O-$, $-OCH_2-$, $-NHCH_2$, or $-CH_2NH-$; |
| n | is zero or an integer from 1 to 3; and |
| p and q | are each independently an integer from 1 to 4, with the provisos that |

(a) when $R^5$ and $R^6$ form a linking group, A is not

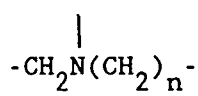

and

(b) when A represents a group

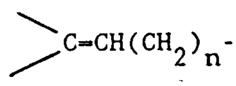

and n is 1, $R^1$ is methyl, $NR^3R^4$ is pyrrolidino both hydrogen then $R^2$ is not hydrogen

or $-CH=CHCO_2H$.

In the compounds of formula (I) at least one of $R^1$ and $R^2$ preferably represents a halogen atom.

A $C_{1-4}$ alkyl or alkoxy group may be straight or branched and may be for example methyl, ethyl, propyl, isopropyl, butyl, methoxy, ethoxy, propoxy, isopropoxy or butoxy.

A $C_{1-7}$ bivalent aliphatic hydrocarbon group is preferably a $C_{1-4}$ alkyl or $C_{2-4}$ alkenyl residue.

A heterocyclic group may be for example pyrrolidino, piperidino, piperazino, ($C_{1-4}$ alkyl) piperazino or morpholino.

Compounds of general formula (I) and their pharmaceutically acceptable salts will hereinafter also be referred to as "potentiating agents".

The present invention also provides the use of compounds of formula (I) and salts thereof in the manufacture of a medicament for enhancing the therapeutic effect of an antineoplastic agent.

Another aspect of the present invention is the use of a compound of formula (I) or a pharmaceutically acceptable salt thereof to increase the sensitivity of a tumor to an antineoplastic agent when the tumor is resistant to the antineoplastic agent by administering to the subject harboring the resistant tumor a potentiating agent concurrently with an antineoplastic agent. Resistance to the antineoplastic agent may (a) be an intrinsic property of the tumor or (b) develop in response to prior treatment with the same antineoplastic agent or another antineoplastic agent capable of selecting for multi-drug resistance.

Another aspect of the present invention is the use of a compound of formula (I) or a pharmaceutically acceptable salt thereof to selectively inhibit the growth of tumor cells in a subject in need of such treatment by concurrently administering to the subject an antineoplastic agent and a potentiating agent. The potentiating agent is administered in an amount effective to (a) reduce the amount of the antineoplastic agent required to achieve the same growth inhibiting effect on the tumor cells by the antineoplastic agent achieved without the concurrent administration of the potentiating agent; or (b) inhibit the development of multiple drug resistance in the tumor cells after treatment with the antineoplastic agent over time.

Another aspect of the present invention is the use of a compound of formula (I) or a pharmaceutically acceptable salt thereof to inhibit multiple drug resistance in a subject in need of such treatment by administering to the subject a potentiating agent in an amount effective to combat multiple drug resistance.

The present invention also provides the use of a compound of formula (I), or a pharmaceutically acceptable salt thereof, for the manufacture of a medicament for inhibiting multiple drug resistance in tumors, increasing the sensitivity of a resistant tumour to an antineoplastic agent and/or selectively inhibiting the growth of tumour cells.

A preferred group of compounds for use according to the present invention is that of formula (II) below and pharmaceutically acceptable salts thereof:

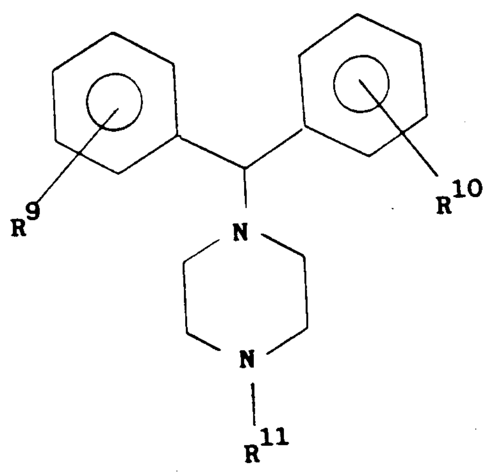

(II)

wherein

$R^9$ is halogen (e.g., chlorine or bromine);

$R^{10}$ is hydrogen, halogen (e.g., chlorine, bromine), $C_{1-4}$ alkoxy (e.g., $-OCH_3$), $-OCH_2C_6H_5$, or $-CH=CHCO_2R^{12}$, wherein $R^{12}$ is $C_{1-4}$ alkyl, preferably ethyl; and

$R^{11}$ is $C_{1-4}$ alkyl e.g. $-CH_3$, $-CH_2CH=CH_2$ or $-CH_2C_6H_4CH_3$-

Particularly preferred compounds of formula (II) include:

(A) 1-(3-Bromo-4'-chlorobenzhydryl)-4-methylpiperazine;

(B) (E)-Ethyl 3-(4-chloro-α-(4-methyl-1-piperazinyl)-benzyl)cinnamate;
(C) 1-(3-Benzyloxy-4'-chlorobenzhydryl)-4-methylpiperazine;
(D) 1-(3-Benzyloxy-4'-chlorobenzhydryl)-4-(3-methylbenzyl)piperazine;
(E) 1-(2-Bromo-4'-chlorobenzhydryl)-4-methylpiperazine;
(F) 1-(α-(4-Bromophenyl)-3-methoxybenzyl)-4-methylpiperazine; and
(G) 1-[α-(4-Chlorophenyl)-3-methoxybenzyl]-4-allylpiperazine;

and pharmaceutically acceptable salts thereof, especially the dihydrochloride salts.

Another preferred group of compounds for use according to the present invention is that of formula (III) below and pharmaceutically acceptable salts thereof:

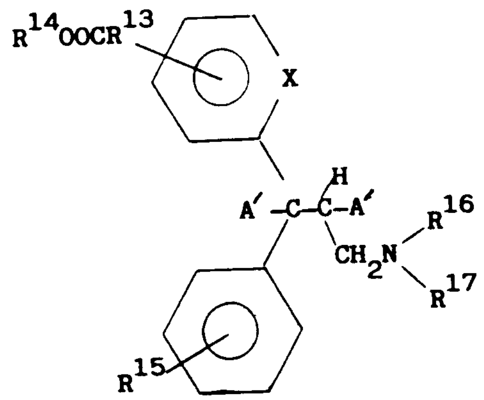

(III)

where $R^{13}$ is a $C_{1-7}$ bivalent aliphatic hydrocarbon group (e.g. a $C_{1-3}$ alkyl or $C_{2-4}$ alkenyl group) or a single bond;
$R^{14}$ is $C_{1-4}$ alkyl;
$R^{15}$ is hydrogen, halogen (e.g. bromo, chloro or fluoro), $C_{1-4}$ alkoxy, $C_{1-4}$ alkyl or $C_{1-4}$ alkyl substituted by 1 to 3 halogen atoms;
$R^{16}$ and $R^{17}$ which may be the same or different each represent a hydrogen atom or a $C_{1-4}$ alkyl group or together with the nitrogen atom to which they are attached form a 4 to 6 membered saturated heterocyclic group (e.g. pyrrolidino, piperidino or morpholino); and
each A' represents a hydrogen atom or -A'C-CA'- represents -C = C-.

Preferred compounds of formula (III) are those of formula (IIIA)

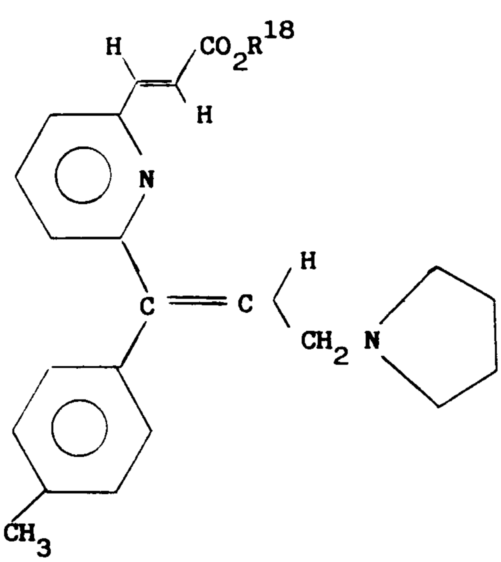

(IIIA)

wherein $R^{18}$ is $C_{1-4}$ alkyl (e.g., methyl or ethyl, preferably ethyl).

Particularly preferred compounds of formula (III) include:-:

(J) Ethyl (E)-3-{6-[3-pyrrolidino-1-(4-tolyl)prop-1E-enyl]-2-pyridyl)acrylate oxalate;
(E)-Isopropyl 3-(6-(E)-3-(1-pyrrolidinyl-1-(4-tolyl)-1-propenyl)-2-pyridyl)acrylate; and
(E)-Butyl 3-(6-(E)-3-(1-pyrrolidinyl-1-(4-tolyl)-1-propenyl)-2-pyridyl)acrylate.

These compounds are believed to be novel and form a further feature of the present invention. These and other compounds of formula (III) may be prepared by the general methods disclosed in US Patent No. 4,650,807 and European Patent No. 85959.

Another preferred group of compounds for use according to the present invention is that of formula (IV) below and pharmaceutically acceptable salts thereof:

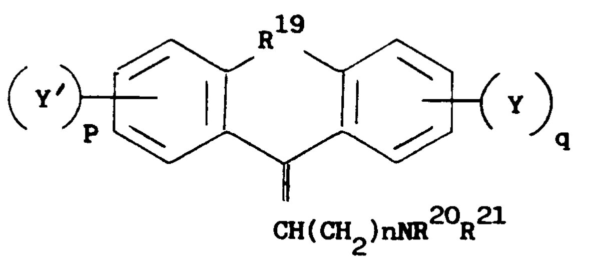

(IV)

wherein:
$R^{19}$ is $-CH_2CH_2-$, $-CH_2O-$, $-OCH_2-$, $-NHCH_2-$, or $-CH_2NH-$;
Y is hydrogen or halogen (e.g., fluorine, chlorine, bromine) attached at the 1, 2, 3 or 4 position;
Y' is hydrogen or halogen (e.g., fluorine, chlorine, bromine) attached at the 7, 8, 9 or 10 position;
n is 0 to 3;
each of p and q is 1 to 4; and
$R^{20}$ and $R^{21}$ independently are hydrogen, or $C_{1-4}$ alkyl, or together with the nitrogen atom form a nitrogen-containing heterocyclic ring having four to six ring members.

Preferably, in the compounds of formula (IV):
$R^{19}$ is $-CH_2CH_2-$, $-CH_2O-$, or $-OCH_2-$;
p and q are 1;
n is 1 or 2; and
$R^{20}$ and $R^{21}$ independently are hydrogen, or $C_{1-4}$ alkyl, or taken together with the nitrogen atom form pyrrolidino.

Preferred compounds of formula (IV) include:
(K) (Z)-3-(2-Bromo-6,11-dihydrodibenz[b,e]oxepin-11-ylidene)-N,N-dimethylpropylamine trihydrochloride;
(L) (E)-3-(2-Bromo-6,11-dihydrodibenz[b,e]oxepin-11-ylidene)-N,N-dimethylpropylamine 3/2 hydrochloride;
(M) (Z)-3-(9-Bromo-6,11-dihydrodibenz[b,e]oxepin-11-ylidene)-N,N-dimethylpropylamine;
(N) (Z)-3-(4-Bromo-6,11-dihydrodibenz[b,e]oxepin-11-ylidene)-N,N-dimethylpropylamine;
(O) (Z)-3-(3-Bromo-6,11-dihydrodibenz[b,e]oxepinylidene)-N,N-dimethylpropylamine;
(P) (Z)-1-[2-(2-Bromo-6,11-dihydrodibenz[b,e]oxepin-11-ylidene)ethyl]pyrrolidine;
(Q) (Z)-1-[3-(2-Bromo-6,11-dihydrodibenz[b,e]oxepin-11-ylidene)propyl]pyrrolidine;
(R) (Z)-1-[2-(6,11-Dihydrodibenz[b,e]oxepin-11-ylidene)ethyl]pyrrolidine;
(S) (Z)-3-(2-Bromo-6,11-dihydrodibenz[b,e]oxepin-11-ylidene)-N,N-diethylpropylamine;
(T) 1-[2-(4-Bromo-6,11-dihydrodibenz[b,e]oxepin-11-ylidine)-ethyl]pyrrolidine;
(U) (E)-1-[3-(2-Bromo-6,11-dihydrodibenz[b,e]oxepin-11-ylidene)propyl]pyrrolidine;
(V) (E)/(Z)-3-(8-Bromo-6,11-dihydrodibenz[b,e]oxepine-11-ylidene)-N,N-dimethylpropylamine;
(W) (Z)-1-[3-(9-Bromo-6,11-dihydrodibenz[b,e]oxepin-11-ylidene)propyl]pyrrolidine;
(X) (E)-3-(2-Bromo-6,11-dihydrodibenz[b,e]oxepin-11-ylidene)-N,N-diethylpropylamine;
(Y) (Z)-1-[3-(2,9-dibromo-6,11-dihydrodibenz[b,e]oxepin-11-ylidene)propyl]pyrrolidine;
(Z) (E)-3-(2-fluoro-6,11-dihydrodibenz[b,e]oxepin-11-ylidine)-N,N-dimethylpropylamine;
(AA) (Z)-3-(2-fluoro-6,11-dihydrodibenz[b,e]oxepin-11-ylidene)-N,N-dimethylpropylamine;
(AB) (E)-1-[3-(2,9-dibromo-6,11-dihydrodibenz(b,e]oxepin-11-ylidene)propyl]pyrrolidine;
(AC) (E)-1-[3-(2-fluoro-6,11-dihydrodibenz[b,e]oxepin-11-ylidene)propyl]pyrrolidine;
(AD) (Z)-1-[3-(2-fluoro-6,11-dihydrodibenz[b,e]oxepin-11-ylidene)propyl]pyrrolidine;
(AE) (Z)-3-(2,9-Dibromo-6,11-dihydrodibenz[b,e]oxepin-11-ylidene)-N,N-dimethylpropylamine;
(AF) (E)-3-(2,9-Dibromo-6,11-dihydrodibenz[b,e]oxepin-11-ylidene)-N,N-dimethylpropylamine 70% and (Z)-isomer 30%; and

(AG) doxepin;

and pharmaceutically acceptable salts thereof.

Certain compounds of formula (IV) are believed to be novel and/or have not previously been described as having any therapeutic utility. These compounds and their use as therapeutic agents form a further aspect of this invention, as set forth in more detail hereinafter.

A further preferred group of compounds for use according to the present invention is that of formula (V) below and pharmaceutically acceptable salts thereof:

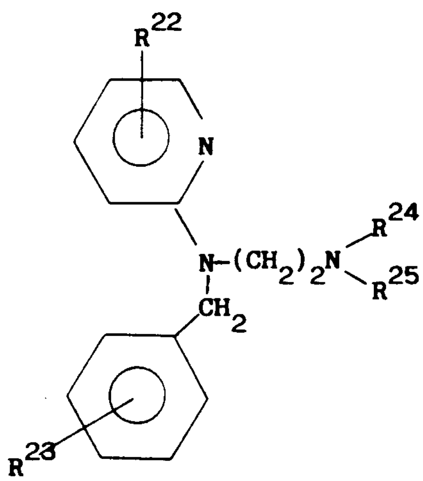

(V)

wherein $R^{22}$ is halogen (e.g., chlorine, bromine), -COOH, -CH = CH-COOH, $-CH_2COOH$, or $-(CH_2)_2COOH$;

$R^{23}$ is hydrogen, halogen (e.g., chlorine, bromine), $C_{1-4}$ alkoxy, or $C_{1-4}$ alkyl; and

$R^{24}$ and $R^{25}$ are the same or different and are each hydrogen or $C_{1-4}$ alkyl, or $NR^{24}R^{25}$ is pyrrolidino.

Preferred compounds of Formula (V) are those compounds of formula (VI):

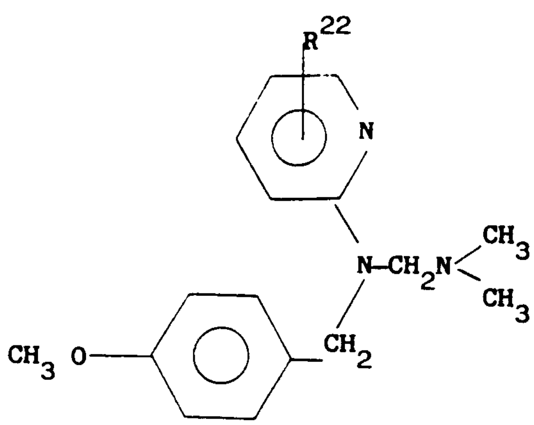

(VI)

wherein $R^{22}$ is halogen, -COOH, -CH = CH-COOH, $CH_2COOH$, or $-(CH_2)_2COOH$

Preferred compounds of formula (VI) include:

(AH) (E)-3-(6-(N-(2-Dimethylaminoethyl)-4-methoxybenzyl-amino)-2 pyridyl)acrylic acid; and

(AI) N-(5-Bromo-2-pyridyl)-N-(4-methoxybenzyl)-N',N'-dimethyl-1,2-ethanediamine; and its hydrobromide and pharmaceutically acceptable salts thereof.

Compounds (AH) and (AI) can be made in the manner described in U.S. Patent No. 4,590,199 to Coker and Findlay (see Examples 1 and 2 therein).

A preferred category of multiple drug resistant tumor cells to be treated by the method of the present invention are multiple drug resistant cells characterized by the multidrug transporter - mediated pumping of antineoplastic agents out of the tumor cells. The multidrug transporter protein is described in M. Gottesman and I. Pastan, supra. Thus, tumor cells treated by the present invention are preferably those characterized by (a) the expression of the multidrug transporter protein at high levels, or (b) the ability to express the multidrug transporter protein upon selection by an antineoplastic agent.

Exemplary of tumor cells which express the multidrug transporter at high levels (intrinsically resistant cells) are adenocarcinoma cells, pancreatic tumor cells, carcinoid tumor cells, chronic myelogenous leukemia cells in blast crisis, and non-small cell lung carcinoma cells.

Exemplary of tumor cells having the ability to express the multidrug transporter protein upon selection by an antineoplastic agent are neuroblastoma cells, pheochromocytoma cells, adult acute lymphocytic leukemia cells, adult acute nonlymphocytic leukemia cells, nodular poorly differentiated lymphoma cells, breast cancer cells and ovarian cancer cells.

A preferred group of tumor cells for treatment in the present invention are the adenocarcinomas, including adenocarcinomas of adrenal, kidney, liver, small intestine and colon tissue, with kidney adenocarcinoma cells particularly preferred.

Preferred antineoplastic agents for use in the present invention are those to which multidrug transporter - mediated multiple drug resistant cells develop resistance. Exemplary of such antineoplastic agents are vinca alkaloids, epipodophyllotoxins, anthracycline antibiotics, actinomycin D, plicamycin, puromycin, gramicidin D, taxol, colchicine, cytochalasin B, emetine, maytansine, and amsacrine (or "mAMSA"). Preferred are vinca alkaloids, epipodophyllotoxins, anthracyclene antibiotics, actinomycin D, and plicamycin.

The vinca alkaloid class is described in Goodman and Gilman's The Pharmacological Basis of Therapeutics, 1277-1280 (7th ed. 1985) (hereafter "Goodman and Gilman"). Exemplary of vinca alkaloids are vincristine, vinblastine, and vindesine.

The epipodophyllotoxin class is described in Goodman and Gilman, supra at 1280-1281. Exemplary of epipodophyllotoxins are etoposide, etoposide orthoquinone, and teniposide.

The anthracycline antibiotic class is described in Goodman and Gilman, supra at 1283-1285. Exemplary of anthracycline antibiotics are daunorubicin, doxorubicin, mitoxantraone, and bisanthrene. Daunorubicin and doxorubicin are preferred.

Actinomycin D, also called Dactinomycin, is described in Goodman and Gilman, supra at 1281-1283. Plicamycin, also called mithramycin, is described in Goodman and Gilman, supra at 1287-1288.

The phrase "concurrently administering," as used herein, means that the antineoplastic agent and the potentiating agent are administered either (a) simultaneously in time (optionally by formulating the two together in a common carrier), or (b) at different times during the course of a common treatment schedule. In the latter case, the two compounds are administered at times sufficiently close for the potentiating agent to enhance the selective growth-inhibiting action of the antineoplastic agent on the tumor cells.

Subjects to be treated by the method of the present invention include both human and animal (e.g., dog, cat, cow, horse) subjects, and are preferably mammalian subjects.

The potentiating agent is administered in an amount effective to enhance the efficacy of the antineoplastic agent. The potentiating agent is preferably administered in a total amount per day of not more than about 50 mg/kg body weight, more preferably not more than about 25 mg/kg, and most preferably not more than about 5 mg/kg. With respect to minimum dose, the potentiating agent is preferably administered in a total amount per day of at least about .01 mg/kg, more preferably at least about .1 mg/kg, and most preferably at least about 1 mg/kg. The potentiating agent may be administered once or several times a day.

As noted above, the compounds of Formula (I) may be administered per se or in the form of a pharmaceutically acceptable salt. When used in medicine, the salts of the compounds of Formula (I) should be both pharmacologically and pharmaceutically acceptable, but non-pharmaceutically acceptable salts may conveniently be used to prepare the free active compound or pharmaceutically acceptable salts thereof and are not excluded from the scope of this invention. Such pharmacologically and pharmaceutically acceptable salts include, but are not limited to, those prepared from the following acids: hydrochloric, hydrobromic, sulphuric, nitric, phosphoric, maleic, acetic, salicylic, p-toluenesulfonic, tartaric, citric, isethionic, methanesulphonic, formic, malonic, succinic, naphthalene-2-sulphonic and benzenesulphonic. Also, pharmaceutically acceptable salts can be prepared as alkaline metal or alkaline earth salts, such as sodium, potassium or calcium salts of the carboxylic acid group. Thus, the present invention also provides pharmaceutical formulations, both for veterinary and for human medical use, which comprise the potentiating agent together with one or more pharmaceutically acceptable carriers thereof and optionally any other therapeutic ingredients. The carrier(s) must be pharmaceutically acceptable in the sense of being compatible with the other ingredients of the formulation and not unduly deleterious to the recipient thereof.

Pharmaceutical formulations of the present invention may optionally include an antineoplastic agent, preferably an agent as described above. Such a formulation is useful for concurrently administering an antineoplastic agent and the potentiating agent in a method as described above.

The formulations include those suitable for oral, rectal, topical, nasal, ophthalmic or parenteral (including subcutaneous, intramuscular and intravenous) administration. Formulations suitable for oral or parenteral administration are preferred.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier, a finely divided solid carrier, or both, and then, if necessary, shaping the product into desired formulations.

Formulations of the present invention suitable for oral administration may be presented as discrete units such as capsules, cachets, tablets or lozenges, each containing a predetermined amount of the potentiating agent as a powder or granules; or a suspension in an aqueous liquor or non-aqueous liquid such as a syrup, an elixir, an emulsion or a draught.

A tablet may be made by compression or molding, optionally with one or more accessory ingredients. Compressed tablets may be prepared by compressing in a suitable machine, with the active compound being in a free-flowing form such as a powder or granules which is optionally mixed with a binder, disintegrant, lubricant, inert diluent, surface active agent or dispersing agent. Molded tablets comprised of a mixture of the powdered active compound with a suitable carrier may be made by molding in a suitable machine.

A syrup may be made by adding the active compound to a concentrated aqueous solution of a sugar, for example sucrose to which may also be added any accessory ingredient(s). Such accessory ingredient(s) may include flavorings, suitable preservatives, an agent to retard crystallization of the sugar, and an agent to increase the solubility of any other ingredient, such as a polyhydric alcohol, for example glycerol or sorbitol.

Formulations suitable for parenteral administration conveniently comprise a sterile aqueous preparation of the active compound, which is preferably isotonic with the blood of the recipient.

Nasal spray formulations comprise purified aqueous solutions of the active compound with preservative agents and isotonic agents. Such formulations are preferably adjusted to a pH and isotonic state compatible with the nasal mucous membranes.

Formulations for rectal administration may be presented as a suppository with a suitable carrier such as cocoa butter, or hydrogenated fats or hydrogenated fatty carboxylic acids.

Ophthalmic formulations are prepared by a similar method to the nasal spray, except that the pH and isotonic factors are preferably adjusted to match that of the eye.

Topical formulations comprise the active compound dissolved or suspended in one or more media such as mineral oil, petroleum, polyhydroxy alcohols or other bases used for topical pharmaceutical formulations. The addition of other accessory ingredients, vide infra, may be desirable.

In addition to the aforementioned ingredients, the formulations of this invention may further include one or more accessory ingredient(s) selected from diluents, buffers, flavoring agents, binders, disintegrants, surface active agents, thickeners, lubricants, preservatives (including antioxidants) and the like.

Certain of the compounds of formula (IV) above have been disclosed for example in US Patent No. 3420851 as having therapeutic utility, principally as psychotherapeutic agents. Others have been disclosed for example in US Patent No. 4871865 as intermediates, with no therapeutic utility. However, a number of the compounds of formula (IV) above are believed to be novel.

The present invention thus further provides novel compounds of formula (IVa) as defined for formula (IV) above with the provisos that

a) when $R^{19}$ is $-CH_2CH_2-$, $-CH_2O-$ or $-OCH_2-$, p and q are each 1 and one of Y and Y' is halogen, the other is not hydrogen; and

b) when $R^{19}$ is $-CH_2CH_2-$ or $-CH_2O-$, p and q are both 1 and each of Y and Y' is hydrogen or halogen, then n is not 2.

The present invention also provides pharmaceutical formulations containing a novel compound of formula (IVa) or a pharmaceutically acceptable salt thereof, and processes for preparing novel compounds of formula (IVa) as described in more detail hereinafter.

In a further aspect the present invention provides, for use as a therapeutic agent a compound of formula (IVb) as defined for formula (IV) above or a pharmaceutically acceptable salt thereof with the proviso that:
when $R^{19}$ is $-CH_2CH_2-$ or $-CH_2O-$, p and q are both 1 and each of Y and Y' is hydrogen or halogen, then n is not 2.

Pharmaceutical compositions comprising such compounds are also within the scope of the present invention. The compounds of formulae (IVa) and (IVb) may be formulated as described above.

In addition to their use in enhancing the therapeutic effect of antineoplastic agents the compounds of formulae (IVa) and (IVb) are useful as antihistaminic and antiasthmatic agents.

Novel compounds of formula (IV) may be prepared in the manner described below, or are prepared by procedures which will be apparent to those skilled in the art.

A compound of Formula (IV) may be prepared via the Wittig method (see, e.g., U.S. Patent Nos. 3,354,155 and 3,509,175) by reaction of a compound of Formula (VIII) below with a Wittig reagent.

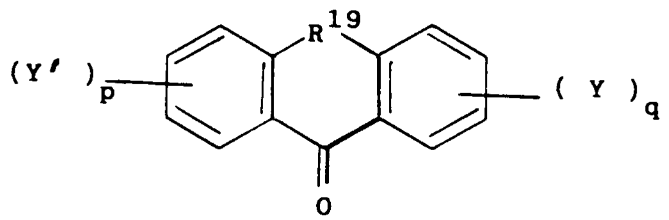

(VIII)

The Wittig reagent, $(C_6H_5)_3P=CH(CH_2)nNR^{20}R^{21}$, is prepared by reacting a compound of the formula $(C_6H_5)_3PCH_2(CH_2)nNR^{205}R^{21}$, with a strong base, such as sodium hydride or $C_{1-6}$ alkyl lithium, in a suitable inert solvent, such as tetrahydrofuran or dimethoxyethane, at or near room temperature.

Compounds of formula (VIII) may be prepared for example using the general method described in US Patent No. 4,871,865.

A compound of Formula (IV) also may be prepared via the Grignard reaction (see, e.g., Belg Patent No. 623,259) in which a Grignard reagent, i.e., $R^{20}R^{21}NCH_2CH_2CH_2Mg$ Hal, where Hal is a halogen atom, is reacted with a compound of Formula (VIII), followed by dehydration with a strong acid.

Compounds of Formula (IV) may also be prepared from other compounds of Formula (IV) by the exchange of halogens thereon. One method for halogen exchange is the Finkelstein halide interchange reaction. Usually, the interchange of a chlorine or bromine of aromatic rings with iodine is carried out by use of a metal iodide such as cuprous iodide, or with fluorine by use of a metal fluoride such as silver(I) fluoride in a solvent such as pyridine or quinoline or N,N-dimethylformamide with heat. A bromo compound may be converted to its corresponding chloro analogue by either use of a metal chloride such as cuprous chloride or lithium chloride in a solvent such as N,N-dimethylformamide, picoline or dimethylsulfoxide with heat (100-103 ° C), or by chlorine in radical form by radiation at temperatures of 30-50 ° C. A bromo compound may also be converted to its iodo analogue by exchanging the bromine for a metal using a metal alkyl such as n-butyllithium in a ethereal solvent such as diethyl ether or tetrahydrofuran at low temperature (e.g., -78 ° C), followed by reacting the metalated species with iodine cold or at room temperature or with heat to accomplish the iodination.

Compounds of Formula (IV) may also be prepared from compounds of Formula (IX), wherein $R^{19}$, $R^{20}$, and $R^{21}$ are as given above, by direct halogenation.

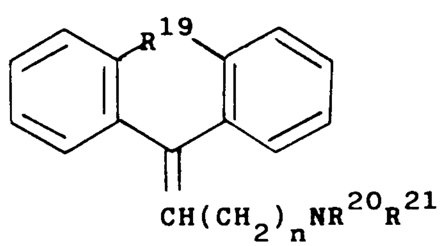

(IX)

Halogenation is preferably carried out with chlorine or bromine in an inert solvent such as acetic acid, methanol, or carbon tetrachloride. In nonpolar solvents, the reaction may be catalyzed by added reagents such as hydrogen chloride and trifluoroacetic acid. The catalytic effect of trace amounts of Lewis acids added to the reaction solution may be employed, such as zinc chloride or ferric chloride in chlorinations and metallic iron, which generates FeBr3 often added to Bromination mixtures. See, e.g., F. Carey and R. Sundberg, Advanced Organic Chemistry Part B: Reactions and Synthesis, 260-63 (1977).

The new compounds of formula (IV) and new pharmaceutical compositions containing them have antiallergic activity and may be used for the same indications as clinically used antiasthmatic compounds, namely to help to control bronchoconstriction or bronchospasm characteristic of allergic asthma and exercise induced asthma and the symptoms of bronchoconstriction and bronchospasm resulting from acute or chronic bronchitis. The compounds and compositions are believed to inhibit the release of autocoids (i.e., histamine, serotonin and the like) from mast cells and to inhibit directly the antigen-induced production of histamine. Thus, they may be classified as mast cell stabilizers with antihistaminic action.

The new compounds and new pharmaceutical compositions of this invention have antihistamine activity, and may be used for the same indications as clinically used antihistamines, namely to relieve detrimental symptoms (caused by histamine release) of nasal stuffiness due to colds and vasomotor rhinitis and for the symptomatic control of allergic conditions including nasal allergy, perennial rhinitis, urticaria, angloneurotic oedema, allergic conjunctivitis, food allergy, drug and serum reactions, insect bites and stings and desensitizing reactions. The compounds and compositions may also be used in conditions responsive to their antipruritic activity including allergic dermatoses, neurodermatitis, anogenital pruritus, and pruritus of non-specific origin such as eczema, and of specific cause such as chickenpox, photosensitivity and sunburn. The present invention therefore provides a method for the symptomatic treatment of allergic conditions by the administration of an effective amount of the new compounds and compositions. The present invention also provides a method for the antagonism of endogenously released histamine by the administration of an effective amount of the new compounds and compositions.

The amount of active compound required for antiasthmatic or antihistaminic use will vary with the compound chosen, the route of administration and the condition of mammal undergoing treatment, and is ultimately at the discretion of the physician. A suitable oral dose of the active compound for a mammal is in the range of from 0.003 to 1.0 milligram per kilogram body weight per day; preferably from 0.04 to 0.24 milligram per kilogram.

The desired daily dose is preferably presented as from one to six sub-doses administered at appropriate intervals throughout the day as needed. Where three sub-doses are employed, each will preferably lie in the range of from 0.014 to 0.08 milligrams per kilogram body weight; for example, a typical sub-dose of such a compound for a human recipient is between 1 and 20 milligrams, for example, 4 or 8 milligrams.

The following Examples are provided to illustrate the present invention, and should not be construed as limiting thereof. Temperatures are given in degrees Celsius unless otherwise indicated.

Unless otherwise stated chromatography was carried out on Waters Prep 500 silica gel.

The following abbreviations are used in the Examples:

THF - tetrahydrofuran
DMF - N,N-dimethylformamide.

EXAMPLE 1

(E)-Ethyl 3-(4-chloro-α-(4-methyl-1-piperazinyl)benzyl)cinnamate (Compound B)

1-(3-Bromo-4'-chlorobenzhydryl)-4-methylpiperazine (see U.S. Patent No. 4,757,074) (1.00 g, 2.63 mmole) was dissolved in 12 ml of dry THF under nitrogen and cooled to -78° C. A solution of 1.1 M n-butyllithium in hexane (2.4 ml) was added dropwise and the reaction was stirred for ten minutes. DMF (0.25 ml, 3.2 mmole) was added in one portion and the reaction was allowed to warm to -40° C. Hydrochloric acid (1.0 M, 20 ml) was added and the reaction was warmed to room temperature. The solution was washed with 20 ml of diethyl ether and the ethereal wash was discarded. The aqueous solution was made basic with aqueous sodium hydroxide and extracted with two 15 ml portions of chloroform. The combined chloroform extracts were dried over sodium sulfate and the solvent removed to give 738 mg of crude 1-(4-chloro-3'-formylbenzhydryl)-4-methylpiperazine as a dark oil.

Triethylphosphonoacetate (0.59 ml, 3.0 mmole) was added dropwise to a flask containing 50% sodium hydride dispersion in oil (128 mg, 2.7 mmole) and 3.5 ml of dry THF under nitrogen. The reaction was stirred at room temperature for 15 minutes or until hydrogen evolution ceased. The 738 mg of crude aldehyde from above (approximately 2.2 mmole) was dissolved in 9 ml of dry THF and added to the reaction. After stirring overnight at room temperature, the reaction was diluted with 15 ml of 1.0 M hydrochloric acid and washed with 20 ml of diethyl ether. The aqueous layer was made basic with aqueous sodium hydroxide and extracted with two 15 ml portions of chloroform. The combined chloroform extracts were dried over sodium sulfate and the solvent was removed to give 890 mg of crude cinnamate ester which was purified by silica gel chromatography with dichloromethane/ethanol/triethylamine (100:0.5:0.1) to give 560 mg of an oil. The product was converted to the dihydrochloride salt with ethanolic hydrochloric acid and the salt was crystallized from diethyl ether to give a hygroscopic white salt. Drying under vacuum gave 412 mg (32% overall) of (E)-ethyl 3-(4-chloro-α-(4-methyl-1-piperazinyl)benzyl)cinnamate as white crystals, mp. 165-172° C., calc. for $C_{23}H_{27}ClN_2O_2$ 2HCl $H_2O$: C, 56.39; H, 6.38; N, 5.72; Cl, 21.71. Found: C, 56.40; H, 6.39; N, 5.72; Cl, 21.67.

EXAMPLE 2

1-(3-Benzyloxy-4'-chlorobenzhydryl)-4-(3-methylbenzyl)piperazine (Compound D)

α-Bromo-m-xylene (10 ml, 74 mmole) was added to a solution of 75 g (11.8 equiv.) of anhydrous piperazine in 500 ml of benzene and the reaction was heated to reflux for two hours. After cooling to room temperature, the reaction was diluted with 300 ml of diethyl ether and washed with 500 ml of 0.2 M sodium hydroxide followed by two 500 ml portions of water. The organic solution was dried over sodium sulfate and the solvent removed to give 11.0 g (78%) of crude 1-(3-methylbenzyl)piperazine as a colorless oil.

The crude piperazine derivative from above (11.0 g, 56 mmole) was dissolved in 150 ml of toluene and added to 19.2 g (56 mmole) of 3-benzyloxy-4'-chlorobenzhydryl chloride (see U.S. Patent No. 4,757,074) and heated to reflux for 40 hours. The reaction was cooled to room temperature, diluted to 600 ml with diethyl ether, and washed with 100 ml of 1.0 M sodium hydroxide followed by three 200 ml portions of water and 100 ml of saturated sodium chloride. The product was dried over sodium sulfate and the solvent removed to give 27.0 g of crude product as a dark oil. The material was purified by chromatography on silica gel with 0.1% triethylamine in dichloromethane to give 14.7 g (53%) of 1-(3-benzyloxy-4'-chlorobenzhydryl)-4-(3-methylbenzyl)piperazine as a dark oil. A 5.0 g portion of the oil was dissolved in 300 ml of 2:1 ethanol methanol and treated with an excess of ethanolic hydrochloric acid to produce the dihydrochloride salt of the product. Addition of 150 ml diethyl ether and cooling provided 1.74 g of the salt as white crystals, mp. 204-208 °C. Calc. for $C_{32}H_{33}ClN_2O$ 2HCl: C, 67.43; H, 6.19; N, 4.91; Cl, 18.66. Found: C, 67.15; H, 6.14; N, 4.87; Cl, 18.58.

## EXAMPLE 3

1-(α-(4-Bromophenyl)-3-methoxybenzyl)-4-methylpiperazine (Compound F)

p-Dibromobenzene (7.08 g, 30.0 mmole) was added to 50 ml of anhydrous THF under nitrogen and the slurry was cooled to -78 °C. A solution of n-butyllithium in hexane (1.67 M, 18.0 ml, 30 mmole) was added dropwise over 15 minutes. After stirring for ten minutes at -78 °C, 3.8 ml (4.1 g, 30 mmole) of m-anisaldehyde was added dropwise over five minutes. After stirring for 15 minutes, the reaction was quenched with 10 ml of saturated aqueous ammonium chloride solution and was allowed to warm to room temperature. The reaction solution was diluted with 300 ml of diethyl ether and washed with three 50 ml portions of 1.0 M aqueous sodium bisulfite, followed by 50 ml of 1.0 M sodium hydroxide, 100 ml of water, and 100 ml of saturated sodium chloride. After drying over magnesium sulfate, the solvent was removed to give 8.5 g (97%) of crude (4-bromophenyl)(3-methoxyphenyl)methanol.

The crude alcohol from above (6.5 g, 22 mmole) was dissolved in 50 ml of dichloromethane, and 2.4 ml (33 mmole) of thionyl chloride was added dropwise at room temperature. The reaction was allowed to stir overnight and the solvent was removed under vacuum. The crude product was redissolved in 50 ml of toluene and the solvent was again removed under vacuum in order to eliminate excess thionyl chloride, providing 6.85 g of crude 4-bromo-3'-methoxybenzhydryl chloride as a dark oil.

The crude benzhydryl chloride from above (6.5 g, 21 mmole) was added to 8.3 g (83 mmole) of 1-methylpiperazine and the reaction was heated to reflux for four hours. After cooling to room temperature, the mixture was dissolved in 100 ml of ethyl acetate and washed with two 150 ml portions of 1.0 M sodium hydroxide, followed by two 150 ml portions of water and 100 ml of saturated aqueous sodium chloride. The solution was dried over magnesium sulfate and the solvent removed to give 6.5 g of crude product as an oil. The product was purified by chromatography on silica gel with dichloromethane:ethanol:triethylamine (100:0.2:0.1) to give a colorless oil. A portion was converted to the dihydrochloride salt with ethanolic hydrochloric acid and precipitated from ethanol with 1:1 hexane:diethyl ether to give 1-(α-(4-bromophenyl)-3-methoxybenzyl)-4-methylpiperazine dihydrochloride as a white powdery solid, mp. 195-197 °C. Calc. for $C_{19}H_{23}BrN_2O$ 2HCl: C, 50.91; H, 5.62; N, 6.25; Total halogen calc. as Cl, 23.73. Found: C, 51.00; H, 5.66; N, 6.23; Total halogen calc. as Cl, 23.78.

## EXAMPLE 4

1-(2-Bromo-4'-chlorobenzhydryl)-4-methylpiperazine (Compound E)

A solution of 23.0 g (120 mmole) of 4-bromochlorobenzene in 150 ml of dry THF was cooled to -78 °C under nitrogen and 78 ml (120 mmole) of 1.55 M n-butyllithium in hexane was added dropwise at a rate to maintain temperature below -60 °C. After stirring an additional 15 minutes, 14.0 ml (120 mmole) of 2-bromobenzaldehyde was added and the reaction was stirred for 15 minutes. The reaction was quenched at -78 °C

with saturated aqueous ammonium chloride and allowed to warm to room temperature. The reaction solution was diluted with 300 ml of diethyl ether and washed with three 75 ml portions of 1.0 M sodium bisulfite, followed by 50 ml of 1.0 M sodium hydroxide, 100 ml of water, and 50 ml of saturated sodium chloride. After drying over magnesium sulfate, the solvent was removed to give 35.3 g (99%) of crude 2-bromo-α-(4-chlorophenyl)benzyl alcohol as a pale yellow oil.

The crude benzhydryl alcohol from above (31.8 g, 107 mmole) was dissolved in 150 ml of dichloromethane and 11.7 ml (1.5 equiv.) of thionyl chloride was added dropwise over ten minutes. The solution was stirred overnight at room temperature and the solvent was removed under vacuum. The crude product was redissolved in 100 ml of toluene and the solvent was again removed under vacuum in order to eliminate excess thionyl chloride, providing crude product as a dark oil. The material was purified by short path distillation (Kugelrohr apparatus, 150°C, 0.5 mm) to give 25.8 g (76%) of (2-bromophenyl)chloro(4-chlorophenyl)methane as a pale yellow oil.

The crude benzhydryl chloride from above (22.5 g, 71 mmole) was added to 32 ml (4 equiv.) of 1-methylpiperazine and heated at 150°C for 20 hours. After cooling to room temperature, the reaction mixture was dissolved in 250 ml of dichloromethane and washed with 20 ml of 1.0 M sodium hydroxide and four 250 ml portions of water. The organic solution was dried with sodium sulfate and the solvent removed to give a black oil. The oil was redissolved in 200 ml of diethyl ether with 100 ml of 1.0 M hydrochloric acid. The layers were separated and the ether layer was extracted with another 100 ml of 0.01 M hydrochloric acid. Combined aqueous layers were made basic with 1.0 M sodium hydroxide and extracted with three 100 ml portions of dichloromethane. The combined dichloromethane extracts were dried over sodium sulfate and the solvent removed to give 13.5 g (50%) of a pale yellow oil. A portion (8.8 g) of the oil was converted to the dihydrochloride salt with excess ethanolic hydrochloric acid and precipitated from ethanol with diethyl ether to give 6.88 g of 1-(2-bromo-4'-chlorobenzhydryl)-4-methylpiperazine dihydrochloride as a white powdery solid, mp. 229-231°C. Calc. for $C_{18}H_{20}BrClN_2$ 2HCl: C, 47.76; H, 4.90; N, 6.19; Total halogen calc. as Cl, 31.33. Found: C, 47.80; H, 4.92; N, 6.17; Total halogen calc. as Cl, 31.32.

EXAMPLE 5

1-(α-(4-Chlorophenyl)-3-methoxybenzyl)-4-allylpiperazine (Compound G)

A solution of 35.0 g (183 mmole) of 4-bromochlorobenzene in 300 ml of dry tetrahydrofuran was cooled to -78°C under nitrogen and 115 ml (183 mmole) of 1.59 M n-butyllithium in hexane was added dropwise at a rate to maintain temperature below -60°C. After stirring an additional 15 minutes, 23.0 ml (183 mmole) of m-anisaldehyde was added dropwise and the reaction was stirred for 15 minutes. The reaction was quenched at -78°C with saturated aqueous ammonium chloride and allowed to warm to room temperature. The reaction solution was diluted with 300 ml of diethyl ether and washed with three 150 ml portions of 1.0 M sodium bisulfite, followed by 100 ml of 1.0 M sodium hydroxide, 100 ml of water, and 50 ml of saturated sodium chloride. After drying over magnesium sulfate, the solvent was removed to give 45.2 g (99%) of crude 4-chloro-α-(3-methoxyphenyl)benzyl alcohol as a pale yellow oil.

The crude benzhydryl alcohol from above (45.2 g, 182 mmole) was dissolved in 400 ml of dichloromethane and 16 ml (1.2 equiv.) of thionyl chloride in 100 ml of dichloromethane was added dropwise. The solution was stirred overnight at room temperature and the solvent was removed under vacuum. The crude product was redissolved in 200 ml of toluene and the solvent was again removed under vacuum in order to eliminate excess thionyl chloride, providing crude product as a dark oil.

The crude benzhydryl chloride from above (approx. 48 g, 180 mmole) was dissolved in 200 ml of toluene with 23 g (1 equiv.) of N-allylpiperazine and the bulk of the toluene was removed under vacuum. Tetramethylethylenediamine (30 ml, approx. 1.1 equiv.) was added to the mixture and the resulting reaction was heated at reflux for 20 hours under nitrogen. The remaining solvent was removed under vacuum. The residue was dissolved in 500 ml of dichloromethane and washed with 250 ml of 1.0 M sodium hydroxide, followed by three 500 ml portions of water. After drying over sodium sulfate, the solvent was removed to give 59.7 g of dark oil. The material was purified by chromatography on silica gel (Waters Prep. 500) with 0.1% triethylamine in dichloromethane to give 28.97 g (45%) of dark oil. The product was converted to its dihydrochloride salt with excess ethanolic hydrochloric acid and precipitated from ethanol with 1:1 hexane:diethyl ether to give 27.6 g of 1-(α-(4-chlorophenyl)-3-methoxybenzyl)-4-allylpiperazine dihydrochloride as a white powder, mp. 215-218°C. Calc. for $C_{21}H_{25}ClN_2O$ 2HCl: C, 58.68; H, 6.33; N, 6.52; Cl, 24.75. Found: C, 58.49; H, 6.37; N, 6.46; Cl, 24.68.

EXAMPLE 6

(E)/(Z)-1-[3-(2-Bromo-6,11-dihydrodibenz[b,e]oxepin-11-ylidene)propyl]pyrrolidine (Compounds U and Q)

Anhydrous 3-(N-pyrrolidinyl)propyltriphenylphosphonium bromide hydrobromide (24 g, 45 mmole) was suspended in 650 ml dry THF and 90 mmole of n-butyl lithium in hexane (1.6 M) was added dropwise at 0°C under a nitrogen atmosphere during a 30-minute period. After an additional ten minutes, 2-bromo-6,11-dihydrodibenz[b,e]oxepin-11-one (10 g, 34.6 mmole), which was prepared as described in U.S. Patent 4,282,365, in 100 mL dry THF was added slowly to the deep red solution and the reaction mixture was then refluxed for 18 hours. The reaction mixture was poured onto ice water, and the mixture was extracted with diethyl ether. The ether layer was concentrated under reduced pressure and the residue was chromatographed on a silica gel column with ethyl acetate/methanol (9:1) to give the pure Z-bromopyrrolidine (1.10 g) as a yellow solid, mp 103-104°C. pmr (DMSO-d6) δ: 7.24-7.35 (m, 6H, aromatic H); 6.74 (d, J=8.4 Hz, 1H, H4); 5.74 (t, 1H, CH=); 5.20 (br s, 2H, $ArCH_2O$); 2.56-2.69 (m, 8H, $CH_2C=$ and 3 N-$CH_2$); 1.81 (m, 4H, 2 $CH_2$).

Analysis: Calculated for $C_{21}H_{22}BrNO$: C, 65.63; H, 5.77; N, 3.64.
Found: C, 65.69; H, 5.80; N, 3.63.

This also offered the pure E-bromopyrrolidine and converted to its hydrochloride salt (0.11 g) as a white solid, mp 203-205°C. pmr (DMSO-d6) δ: 7.29-7.46 (m, 6H, aromatic H); 6.70 (d, J=8.8 Hz, 1H, H4); 6.09 (t, 1H, CH=); 5.20 (br s, 2H, $ArCH_2O$);4.31-1.02 (m, 12H, $3NCH_2$ and $3CH_2$).

Analysis: Calcd. for $C_{21}H_{22}BrNO$ 0.05 $H_2O$ 1.3 HC1: C, 58.30; H, 5.45; N, 3.24.
Found: C, 58.21; H, 5.35, N, 3.24.

EXAMPLE 7

(E)/(Z)-1-[2-(2-Bromo-6,11-dihydrodibenz[b,e]oxepin-11-ylidene)ethyl]pyrrolidine (Compound P)

Anhydrous 2-(N-pyrrolidinyl)ethyltriphenylphosphonium bromide (10 g, 45 mmole), 48 mmole of n-butyl lithium in hexane, and 2-bromo-6,11-dihydrodibenz[b,e]-oxepin-11-one (5 g, 17.3 mmole) were reacted in 300 mL dry THF by the procedure in Example 6. This provided the pure Z-isomer (1.76 g) as a light brown solid, mp 108-109°C. pmr (DMSO-d6) δ: 7.28-7.50 (m, 6H, aromatic H); 6.80 (d, J = 8.8 Hz, 1H, H4); 5.82 (t, 1H, HC=); 5.20 (s, 2H, $ArCH_2O$); 3.29 (m, 2H, $NCH_2C=$); 2.50 (m, 4H, $2NCH_2$); 1.70 (m, 4H $2CH_2$).

Also, the pure (E)-isomer was obtained as a light-brown oil. pmr (DMSO-d6) δ: 7.22-7.51 (m, 6H, aromatic H); 6.79 (d, J = 8.8 Hz, 1H, H4); 6.16 (t, 1H, HC=); 5.40-5.50 (br s, 2H, $ArCH_2O$); 3.0 (m, 2H, $NCH_2C=$); 2.36 (m, 4H, $2NCH_2$); 1.64 (m, 4H $2CH_2$).

Analysis: Calculated for $C_{20}H_{20}BrNO$: C, 64.87; H, 5.44; N, 3.78.
Found: (Z-isomer): C, 64.83; H, 5.49; N, 3.75.
Found (E-isomer): C, 64.79; H, 5.45; N, 3.77.

EXAMPLE 8

(E)/(Z)-3-(2-Bromo-6,11-dihydrodibenz[b,e]-oxepin-11-ylidene)-N,N-diethylpropylamine (Compounds S and X)

Anhydrous 3-(diethylamino)propytriphenylphosphonium bromide hydrobromide (15 g, 28 mmole), 56 mmole of n-butyl lithium in hexane (1.6 M), and 2-bromo-6,11-dihydrodibenz [b,e]-oxepin-11-one (8.1 g, 28 mmole) were reacted in 350 mL dry THF by the procedure in Example 6. This provided a (Z)/(E) (90:10) isomeric product as a brown oil. pmr (DMSO-d6) δ: 7.22-7.51 (m, 6H, aromatic H); 6.78 (d, J = 8.8 Hz, H4 of 90% Z); 6.68 (d, J = 8.8 Hz, H4 of 10% of E); 6.19 (t, CH= of 10% E); 5.73 (t, CH= of 90% Z); 5.20 (br s, 2H, $ArCH_2O$); 2.30-2.60 (m, 8H, 4 $CH_2$); 0.94 (t, 6H, 2 $CH_3$ of 90% Z); 0.88 (t, 6H, 2 $CH_3$ of 10% E).

Analysis: Calculated for $C_{21}H_{24}BrNO$: C, 65.29; H, 6.26; N, 3.63; Br, 20.68.
Found: C, 65.27; H, 6.26; N, 3.60; Br, 20.59.

EXAMPLE 9

(E)/(Z)-3-(2-Fluoro-6,11-dihydrodibenz[b,e]-oxepin-11-ylidene)-N,N-dimethylpropylamine (Compounds AA and Z)

Anhydrous 3-(dimethylamino)propyltriphenylphosphonium bromide hydrobromide (35 g, 0.07 mole), 0.14 mole of n-butyl lithium in hexane (1.6 M), and 2-fluoro-6,11-dihydrodibenz[b,e]oxepin-11-one (12 g,

0.05 mole), which was prepared as described in European patent application No. EP 38,564, were reacted in 800 mL dry THF by the procedure of Example 6. This gave pure Z-isomer (0.67 g) mp 47-48°C. pmr-(DMSO-d6) δ: 6.81-7.37 (m, 7H, aromatic H), 5.73 (t, 1H, CH=), 5.14 (br s, 2H, $ArCH_2O$), 2.37-2.51 (m, 4H, $2CH_2$), 2.09 (s, 7H, $N(CH_3)_2$); and pure E-isomer (1.70 g), mp 44-45°C. pmr (DMSO-d6) δ: 6.68-7.49 (m, 7H, aromatic H), 6.08 (t, 1H, CH=), 5.20 (br s, 2H, $ArCH_2O$), 2.19-2.35 (m, 4H, $2CH_2$), 2.01 (s, 7H, $N(CH_3)_2$).

| | |
|---|---|
| Analysis: | Calculated for $C_{12}H_{20}FNO$ 0.10 $H_2O$ = 299.17: C, 76.26; H, 6.81; N, 4.68. |
| Found: | (Z-isomer): C, 76.20; H, 6.83; N, 4.76. |
| Found: | (E-isomer): C, 76.14; H, 6.80; N, 4.69. |

EXAMPLE 10

(E)/(Z)-1-[3-(2-Fluoro-6,11-dihydrodibenz[b,e]oxepin-11-ylidene)propyl]pyrrolidine (Compounds AC and AD)

Anhydrous 3-(N-pyrrolidinyl)propyltriphenylphosphonium bromide hydrobromide (35 g, 0.07 mole), 0.14 mole of n-butyl lithium in hexane (1.6 M), and 2-fluoro-6,11-dihydrodibenz[b,e]oxepin-11-one (12 g, 0.05 mole), prepared as described in European Patent Application No. EP 38,564, were reacted in 800 mL dry THF by the procedure described in Example 6. This offered pure Z-isomer and was converted to its hydrochloride form (3.0 g), mp 201-202°C. pmr (DMSO-d6) δ: 6.84-7.36 (m, 7H, aromatic H), 5.72 (t, 1H, CH=), 5.18 (br s, 2H, $ArCH_2O$), 3.73-1.95 (m, 12H, $3CH_2$ and $3NCH_2$); and pure E-isomer (0.70 g), mp 77-78°C. pmr (DMSO-d6) δ: 6.69-7.50 (m, 7H, aromatic H), 6.10 (t, 1H, CH=), 5.15 (br s, 2H, $ArCH_2O$), 1.63-2.50 (m, 12H, $3CH_2$ and $3NCH_2$).

| | |
|---|---|
| Analysis: | Calculated for $C_{21}H_{22}FNO$ 0.96 HCl 0.04 $H_2O$: C, 70.23, H, 6.47; N, 3.90; Cl, 9.48. |
| Found: | (Z-isomer): C, 69.96; H, 6.68; N, 3.77; Cl, 9.36. |
| | Calculated for $C_{21}H_{22}FNO$ 0.50 $H_2O$: C, 75.88; H, 6.97; N, 4.21. |
| Found: | (E-isomer): C, 76.03; H, 6.79; N, 4.43. |

EXAMPLE 11

(E)/(Z)-3-(3-Bromo-6,11-dihydrodibenz[b,e]-oxepin-11-ylidene)-N,N-dimethylpropylamine (Compound O)

(a) Methyl 2-(3-bromophenoxymethyl)benzoate

To a mixture of 3-bromophenol (60 g, 0.35 mole) and potassium carbonate (25 g, 0.18 mole) in 250 mL of DMF was added methyl α-bromo-2-toluate (65 g, 0.28 mole). The reaction mixture was stirred at room temperature for 18 hours, then heated on a steam bath for three hours. The mixture was poured into ice water, and the solids were collected by filtration and washed with water to give the crude product. Analytical sample was obtained by recrystallization from methylene chloride/ hexanes, m.p. 84-85°C. pmr ($CDCl_3$) δ: 8.0 (m, 1H, H6), 6.93-7.69 (m, 7H, aromatic H), 5.47 (s, 2H $ArCH_2O$), 3.89 (s, 3H, $CO_2CH_3$).

| | |
|---|---|
| Analysis: | Calculated for $C_{15}H_{13}BrO_3$: C, 56.09; H, 4.08; Br, 24.88. |
| Found: | C, 56.20; H, 4.12; Br, 24.77 |

(b) 2-(3-bromophenoxymethyl)benzoic acid

Methyl 2-(3-bromophenoxymethyl)benzoate (34 g) was refluxed in a mixture of 100 mL of 10% sodium hydroxide and 200 mL of methanol for three hours. The reaction mixture was concentrated under reduced pressure and water was added to the residue. The mixture was then acidified with concentrated hydrochloric acid. Extracting the acidic solution with ethyl acetate and then concentration of the organic layer gave 2-(3-bromophenoxymethyl) benzoic acid (35 g) m.p. 158-159°C. pmr ($CDCl_3$) δ: 8.10 ()m, 1H, $H_6$), 6.84-7.74 (m, 7H, aromatic H), 6.16 (br s, 1H, $CO_2H$), 5.49 (s, 2H, $ArCH_2O$).

| | |
|---|---|
| Analysis: | Calculated for $C_{14}H_{11}BrO_3$: C, 54.74; H, 3.61; Br, 26.02. |
| Found: | C, 54.65; H, 3.61; Br, 26.08. |

(c) 3-bromo-6,11-dihydrodibenz[b,e]oxepin-11-one

A suspension of 2-(3-bromophenoxymethyl)benzoic acid (35 g, 0.11 mole) in 100 mL of trifluoracetic anhydride containing 20 drops of boron trifluoride-ether complex was refluxed for four hours. The mixture was poured into ice water and then extracted with diethyl ether. Concentration of ether solution under

reduced pressure and chromatography of the residue on a silica gel column with hexane/methylene chloride (70:30) gave the pure product (14 g), m.p. 110-112°C. pmr ($CDCl_3$) δ: 8.10 (d, J=9.1 Hz, 1H, $H_1$), 7.90 (dd, J=1.4, 7.6 H, 1H, $H_{10}$) 7.57 (dt, J=1.4, 7.4, 7.4 Hz, 1H, $H_8$), 7.48 (dt, J=1.4, 7.6, 7.6 Hz, 1H, $H_9$), 7.36 (dd, J=1.3, 7.3 Hz, 1H, $H_7$), 7.27 (d, J=1.8 H, 1H, $H_4$), 7.24 (dd, J=1.8, 9.1 Hz, 1H, $H_2$), 5.18 (s, 2H, $ArCH_2O$).

Analysis: Calculated for $C_{14}H_9BrO_2$: C, 58.16; H, 3.14; Br, 27.64.
Found: C, 58.13; H, 3.19; Br, 27.72.

(d) (E)/(Z)-3-(3-Bromo-6,11-dihydrodibenz[b,e]oxepin-11-ylidene)-N,N-dimethylpropylamine

Anhydrous 3-(dimethylamino)propyltriphenylphosphonium bromide hydrobromide (24.5 g, 48.0 mmole), 96 mmole of n-butyl lithium in hexane, and 3-bromo-6,11-dihydrodi-benz[b,e]oxepin-11-one (10 g, 34.6 mmole) were reacted in 580 mL dry THF by the procedure of Example 6. This provided a Z/E(3:1) isomeric mixture of bromoamines (6.0 g). Recrystallization of half of the mixtures (3.0 g) from ethyl acetate gave 1.45 g of Z-isomer of 93% stereoisomeric purity (assayed by $^1$H-NMR) and converted to its hydrochloride salt as a white solid. pmr ($CDCl_3$) δ: 7.23-7.31 (m, 4H, aromatic H), 6.92-7.05 (m, 3H, aromatic H), 5.91 (t, 1H, CH=, 7% E-isomer), 5.60 (t, 1H, CH=, 93% Z-isomer), 5.15 (very br s, 2H, $ArCH_2O$), 3.12 (m, 2H, $CH_2$), 2.99 (m, 2H, $NCH_2$), 2.78 (s, 6H, $NMe_2$, 93% Z-isomer), 2.71 (s, 6H, $NMe_2$, 3% E-isomer).

Analysis: Calculated for $C_{19}H_{20}BrNO$ 1.0 HCl: C, 57.81; H, 5.36; N, 3.55.
Found: C, 57.62; H, 5.33; N, 3.54.

## EXAMPLE 12

### (E)/(Z)-3-(4-Bromo-6,11-dihydrodibenz[b,e]oxepin-11-ylidene)-N,N-dimethylpropylamine (Compound N)

(a) Methyl 2-(2-bromophenoxymethyl)benzoate

Methyl α-bromo-2-toluate (53 g, 0.23 mole) was added to a mixture of 2-bromophenol (40 g, 0.23 mole) and potassium carbonate (42 g, 0.3 mole) in 250 mL of N,N-dimethylformamide. The reaction mixture was stirred at room temperature for 18 hours, then heated on a steam bath for 3.5 hours. The mixture was poured into ice water, and the solids were collected by filtration and washed with water to give the crude product. An analytical sample was obtained by recrystallization from hexane/ethyl acetate (38 g, mp 73-74°C. pmr ($CDCl_3$) δ: 6.58-8.05 (m, 8, aromatic H); 5.48 (s, 2H, $ArCH_2O$); 3.80 (s, 3H, $CO_2CH_3$).

Analysis: Calculated for $Cl_5H_{13}BrO_3$: C, 56.09; H, 4.08; Br, 24.88.
Found: C, 56.12; H, 4.09; Br, 24.85.

(b) 2-(2-bromophenoxymethyl)benzoic acid

Methyl 2-(2-bromophenoxymethyl) benzoate (38 g) was refluxed in a mixture of 100 mL of 10% aqueous sodium hydroxide and 200 mL of methanol for three hours. The reaction mixture was concentrated under reduced pressure and water was added to the residue. The mixture was then acidified with concentrated hydrochloric acid. Extraction of the cooled acidic solution with ethyl acetate and then concentration of the organic layer gave 2-(2-bromophenoxymethyl)benzoic acid (30 g), mp 175-177°C. pmr (CDCl3) δ: 6.79-8.02 (m, 8H, aromatic H); 5.53 (s, 2H, $ArCH_2O$).

Analysis: Calculated for $C_{14}H_{11}BrO_3$: C, 54.74; H, 3.61; Br, 26.02.
Found: 54.64; H, 3.61; Br, 26.14.

(c) 4-Bromo-6,11-dihydrodibenz[b,e]oxepin-11-one

A suspension of 2-(2-bromophenoxymethyl)benzoic acid (18.8 g, 0.06 mole) in 100 mL of trifluoracetic anhydride containing ten drops of boron trifluoride-ether complex was refluxed for four hours. The mixture was poured into ice water and then extracted with diethyl ether. Concentration of the ether solution under reduced pressure gave the pure product (9.2 g), mp 127-130°C. pmr ($CDCl_3$) δ: 7.28-8.19 (m, 76H, aromatic H); 6.99 (t, 1H, H2); 5.30 (s, 2H, $ArCH_2O$).

Analysis: Calculated for $C_{14}H_9BrO_2$: C, 56.16; H, 3.14; Br, 27.64.
Found: C, 58.19; H, 3.18; Br, 27.68.

(d) (E)/(Z)-3-(4-Bromo-6,11-dihydrodibenz[b,e]oxepin-11-ylidene)-N,N-dimethylpropylamine hydrochloride

Anhydrous 3-(dimethylamino)propyltriphenylphosphonium bromide hydrobromide (22.5 g, 0.04 mole) was suspended in 550 mL of dry THF, and 55 mL of a solution of n-butyl lithium in hexane (1.6 M) was added dropwise at 0 ° C under a nitrogen atmosphere during a 30-minute period. After an additional ten minutes, 4-bromo-6,11-dihydrodibenz[b,e]oxepin-11-one (9.2 g, 0.03 mole) in 100 mL of dry THF was added slowly to the deep red solution and the reaction mixture refluxed for 18 hours. The reaction mixture was poured into ice water, and extracted with ethyl ether. The ether layer was concentrated under reduced pressure and the residue was suspended in water, then acidified with 6 N hydrochloric acid. The acidic aqueous layer was washed with hexane then concentrated to give a gummy residue. It was chromatographed on a silica gel column with ethyl acetate/methanol (8:2) to give 5.2 g of product as a mixture of Z/E-isomers (approximately 95:5), mp > 200 ° C (grad. dec.). pmr ($CDCl_3$) δ: 7.56 (dd, 1H, H3); 7.30-7.42 (m, 4H, aromatic H); 7.20 (dd, 1H, H1); 6.92 (t, 1H, H2); 5.72 (t, 1H, CH=); 5.32 (br s, 2H, $CH_2O$); 3.20 (t, 2H, $CH_2N$); 2.68 (m, 2H, $CH_2$); 2.70 (s, 6H, $N(CH_3)_2$); 5% of (E)-isomer by 6.08 (t, 1H, CH=) and 2.64 (s, 6H, N-$(CH_3)_2$).

Analysis: Calculated for $C_{19}H_{20}BrNO$ HCl 0.4 $H_2O$: C, 56.78; H, 5.47; N, 3.48.
Found: C, 56.93; H, 5.30; N, 3.51.

EXAMPLE 13

(E)/(Z)-1-[2-(4-Bromo-6,11-dihydrodibenz[b,e]oxepin-11-ylidene)ethyl]pyrrolidine (Compound T)

Anhydrous 2-(N-pyrrolidinyl)ethyltriphenylphosphonium bromide (20 g, 45 mmole), 45 mmole of n-butyl lithium in hexane (1.6 M), and 4-bromo-6,11-dihydrodi-benz[b,e] oxepin-11-one (10 g, 35 mmole) were reacted in 550 mL dry THF by the procedure in Example 6. This offered a (Z)/(E) (60:40) isomeric product as a light brown oil. pmr (DMSO-$d_6$) δ: 7.28-7.56 (m, 5H, aromatic H); 7.15-7.26 (2 dd, 1H, $H_1$); 6.88 (t, $H_3$ of 60% Z); 6.84 (t, $H_3$ of 40% E); 6.16 (t, CH= of 40% E): 5.86 (t, CH= of 60% Z); 5.30 (br s, 2H, $ArCH_2O$); 2.28-2.54 (m, 6H, $N(CH_2)_3$); 1.66 (m, 4H, $2CH_2$).

Analysis: Calculated for $C_{20}H_{20}BrNO$: C, 64.87; H, 5.44; N, 3.78; Br, 21.58.
Found: C, 64.84; H, 5.46; N, 3.77; Br, 21.68.

EXAMPLE 14

(E)/(Z)-3-(8-Bromo-6, 11-dihydrodiebenz[b,e]oxepin-11-ylidene)-N,N-dimethylpropylamine (Compound V)

(a) 8-Bromo-6,11-dihydrodibenz[b,e]oxepin-11-one

Phenol (8 g, 85 mmole) and potassium carbonate (11.7 g, 85 mmole) in 150 mL of DMF was reacted with methyl 4-bromo-α-bromo-2-toluate (20 g, 65 mmole) by the procedure of Example 11, step a and followed with alkaline hydrolysis by the procedure of Example 11, step b to give the crude 4-bromo-2-(phenoxymethyl)benzoic acid (13 g) which was used without further purification.

The crude 4-bromo-2-(phenoxymethyl)benzoic acid (13 g, 42 mmole) was cyclized in 50 mL of trifluroacetic anhydride containing 1 mL of boron trifluoride ether complex by the procedure of Example 11, step c. The solid was collected by filtration and washed with water to give 11.9 g of the tricyclic ketone, m.p. 125-126 ° C. pmr (CDC3) δ: 8.17-8.30 (m, 1H, $H_1$), 6.99-7.86 (m, 6H, aromatic H), 5.14 (s, 2H, $ArCH_2O$).

Analysis: Calculated for $C_{14}H_9BrO_2$: C, 58.16; H, 3.14; Br, 27.64.
Found: C, 58.15; H, 3.17; Br, 27.73.

(b) (E)/(Z)-3-(8-Bromo-6,11-dihydrodibenz[b,e]oxepin-11-ylidene)-N,N-dimethylpropylamine

Anhydrous 3-(dimethylamino)propyltriphenylphosphonium bromide hydrobromide (24.5 g, 48 mmole), 96 mmole of n-butyl lithium in hexane (1.6 M), and 8-bromo-6,11-dihydrodibenz[b,e]oxepin-11-one (10 g, 34.6 mmole) were reacted in 580 mL dry THF by the procedure of Example 6. This provided an E/Z (1:3.5) isomeric mixture of bromoamines. Recrystallization of the mixture from diethyl ether gave 0.17 g of Z-isomer and 1.8 g of an E/Z (1:4) (assayed by HPLC on C18) isomeric mixture. pmr (Z-isomer) ($CDCl_3$) δ: 7.38-7.44 (m, 2H, $H_7$ and $H_9$); 7.13-7.18 (m, 3H, aromatic H); 6.84-6.93 (m, 2H, $H_2$ and $H_4$); 5.70 (t, 1H, CH=); 5.15 (br s, 2H, $ArCH_2O$); 2.55 (q, 2H, $CH_2$); 2.43 (t, 2H, $NCH_2$); 2.22 (s, 6H, $NMe_2$).

Analysis: Calculated for $C_{19}H_{20}BrNO$: C, 63.70; H, 5.63; N, 3.91.
Found (Z-isomer): C, 63.85; H, 5.65; N, 3.92.

EXAMPLE 15

(E)/(Z)-3-(9-Bromo-6,11-dihydrodibenz[b,e]oxepin-11-ylidene)-N,N-dimethylpropylamine Compound M)

Anhydrous 3-(dimethylamino)propyltriphenylphosphonium bromide hydrobromide (35 g, 69 mmole), 100 mmole of n-butyl lithium in hexane (1.6 M), and 9-bromo-6, 11-dihydrodibenz[b,e]oxepin-11-one (20 g, 69 mmole) were reacted in 750 mL dry THF by the procedure of Example 6. Recrystallization of the mixture from chloroform/carbon tetrachloride gave 6.7 g of the product (Z:E = 93.7) as its hydrochloride salt, melting range 85-88 ° C. pmr ($CDCl_3$) $\delta$: 6.94-7.46 (m, 7H, aromatic), 6.04 (t, CH = of 7% E), 5.64 (t, CH = of 93% Z), 5.15 (br s, 2H, $CH_2O$), 3.07 (m, 4H, $NCH_2$ $CH_2$), 2.75 (s, 6H, $NMe_2$).

Analysis: Calculated for $C_{19}H_{20}BrNO$ 2.0 HCl 0.3 H2O: C, 52.27; H, 5.22; N, 3.21.
Found: C, 52.28; H, 5.23; N, 3.18.

EXAMPLE 16

(E)/(Z)-1-[3-(9-Bromo-6,11-dihydrodibenz[b,e]oxepin-11-ylidene)propyl]pyrrolidine (Compound W)

Anhydrous 3-(N-pyrrolidinyl)propyltriphenylphosphonium bromide hydrobromide (40 g, 0.08 mole), 0.16 mole of n-butyl lithium in hexane (1.6 M), and 9-bromo-6,11-dihydrodibenz[b,e]oxepin-11-one (20 g, 0.07 mole) were reacted in 900 mL dry THF by the procedure described in Example 6. This provided 8.15 g of Z/E (5:1) mixture of 9-bromopyrrolidine which was converted to its hydrochloride salt. The isomeric mixture was washed with hot ethyl acetate to give 6.0 g of the product (Z:E = 93:7) as a white solid, m.p. 214-217 ° C. pmr (DMSO-$d_6$) $\delta$: 6.94-7.56 (m, 6 H, aromatic H), 6.86 (dd, $H_4$ of 93% Z), 6.14 (dd, $H_4$ of 7% E), 6.08 (t, CH = of 7% E), 6.08 (t, CH = of 7% E), 5.76 (t, CH = of 93% Z), 5.20 (br s, 2H $ArCH_2O$), 1.90-3.32 (m, 12H, $6CH_2$).

Analysis: Calculated for $C_{21}H_{22}BrNO$ 1.6 HCl: C, 56.98; H, 5.37; N, 3.16.
Found: C, 56.89,; H, 5.32; N, 3.05.

EXAMPLE 17

(E)/(Z)-3-(2,9-Dibromo-6,11-dihydrodibenz[b,e]oxepin-11-ylidene)-N,N-dimethylpropylamine (Compounds AF and AE)

(a) Methyl 5-bromo-2-(4-bromophenoxymethyl)benzoate

To a mixture of 4-bromophenol (50 g, 0.29 mole) and sodium hydride (14 g, 0.36 mole) in 500 mL of DMF was added methyl $\alpha$-5-dibromo-2-toluate (74 g, 0.24 mole). The reaction mixture was stirred at 40 ° C for 18 hours. The mixture was poured into ice water and the solids were collected by filtration and washed with water to give the product (90 g). Analytical sample was obtained by recrystallization from ethyl acetate, mp 101-102 ° C. pmr (DMSO-$d_6$) $\delta$: 6.90-8.01 (m, 7H, aromatic H), 5.35 (s, 2H, $ArCH_2O$), 3.81 (s, 3H, $CO_2CH_3$).

Analysis: Calculated for $C_{15}H_{12}Br_2O_3$ 0.20 EtoAc: C, 45.43; H, 3.28; Br, 38.26.
Found: C, 45.42; H, 3.10; Br, 38.61.

(b) 5-Bromo-2-(4-bromophenoxymethyl)benzoic acid

Methyl 5-bromo-2-(4-bromophenoxymethyl)benzoate (90 g, 0.23 mole) was refluxed in a mixture of 100 mL of 10% sodium hydroxide and 200 mL of methanol for three hours. The reaction mixture was concentrated under reduced pressure and water was added to the residue. The mixture was acidified with concentrated hydrochloric acid. A solid was precipitated and collected as the acid product (80 g). mp 214-215 ° C. pmr (DMSO-$d_6$) $\delta$: 6.76-8.01 (m, 7H, aromatic H), 5.38 (s, 2H, $ArCH_2O$).

(c) 2,9-Dibromo-6,11-dihydrodibenz[b,e]oxepin-11-one

A solution of 5-bromo-2-(4-bromophenoxymethyl)-benzoic acid (100 g, 0.26 mole), and trifluoroacetic anhydride (160 mL) in one liter of methylene chloride containing 20 drops of boron trifluoride-ether complex was refluxed for three hours. The mixture was poured into ice water and then extracted with diethyl ether. Concentration of ether solution under reduced pressure and chromatography of the residue on a silica gel

column with hexane/ethyl acetate (9:1) gave the ketone (45 g) mp 193-194°C. pmr (DMSO-$d_6$) δ: 8.12 (d, J=2.7 Hz, 1H, $H_{10}$), 7.27-7.91 (m, 4H, aromatic H), 7.10 (d, J=8.8 Hz, 1H, $H_4$), 5.32 (s, 2H, $ArCH_2O$).

| | |
|---|---|
| Analysis: | Calculated for $C_{14}H_8Br_2O_2$: C, 45.69; H, 2.19; Br, 43.42. |
| Found: | 45.49; H, 2.22; Br, 43.27. |

(d) (E)/(Z)-3-(2,9-Dibromo-6,11-dihydrodibenz[b,e]oxepin-11-ylidene)-N,N-dimethylpropylamine

Anhydrous 3-(dimethylamino)propyltriphenylphosphonium bromide hydrobromide (27 g, 0.05 mole), 0.1 mole of n-butyl lithium in hexane (1.6 M), and 2,9-dibromo-6,11-dihydrodibenz[b,e]oxepin-11-one (15 g, 0.04 mole) were reacted in 650 mL dry THF by the procedure of Example 6. This provided an (E)/(Z) isomeric mixture of dibromoamine. Separation of this mixture by a silica gel column (Waters Associates - Prep 50) with ethyl acetate/methanol (9:1) offered the Z-isomer (5.4 g). mp 87-88°C, pmr (DMSO) δ: 7.31-7.54 (m, 5H, aromatic H), 6.78 (d, J=8.9 Hz, 1H, $H_4$), 5.77 (t, 1H, CH=), 5.15 (br s, 2H, $ArCH_2O$), 2.40 (m, 4H, 2 $CH_2$), 2.10 (s, 6H, $N(CH_3)_2$); and a Z/E mixture (30:70) which was converted to its hydrochloride salt by adding one equivalent hydrochloric acid (0.18 g), mp 113-115°C. pmr (DMSO-$d_6$) δ: 7.28-7.78 (m, 5H, aromatic H), 6.82 (d, J=8.8 Hz, $H_4$ of 30% Z), 6.71 (d, J=8.8 Hz, $H_4$ of 70% E), 6.04 (t, CH= of 70% E), 5.76 (t, CH- of 30% Z), 4.90-5.50 (m, 2H, $ArCH_2O$), 2.30-3.19 (m, 10H, 2 $CH_2$ and $N(CH_3)_2$).

| | |
|---|---|
| Analysis: | Calculated for $C_{19}H_{19}Br_2NO$: C, 52.20; H, 4.28; N, 3.20; Br, 36.55. |
| Found | (Z-isomer): C, 52.40; H, 4.40; N, 3.29; Br, 36.39. |
| Calculated for $C_{19}H_{19}Br_2NO$ HCl: | C, 48.18; H, 4.26; N, 2.96. |
| Found (Z/E=30:70): | C, 48.57; H, 4.66; N, 2.68. |

EXAMPLE 18

(E)/(Z)-1-[3-(2,9-Dibromo-6,11-dihydrodibenz[b,e]oxepin-11-ylidene)propyl]pyrrolidine (Compounds AB and Y)

Anhydrous 3-(N-pyrrolidinyl)propyltriphenylphosphonium bromide hydrobromide (47 g, 0.08 mole), 0.20 mole of n-butyl lithium in hexane (1.6 M), and 2,9-dibromo-6,11-dihydrodibenz[b,e]oxepin-11-one (25 g, 0.07 mole) was reacted in one liter of dry THF by the procedure of Example 6. The crude product was chromatographed on a silica gel column with ethyl acetate/ethanol (95:5) to give the product as 98% pure Z-isomer (0.30 g), mp 102-103°C. pmr (DMSO-$d_6$) δ: 7.31-7.54 (m, 5H, aromatic H), 6.79 (d, J=7.8 Hz, $H_4$ of 98% Z), 6.67 (d, J=7.8 Hz, $H_4$ of 2% E), 6.11 (t, CH= of 2% E), 5.78 (t, CH= of 98% Z), 2.34-2.56 (m, 8H, $CH_2$ and $3NHCH_2$), 1.63 (m, 4H, $2CH_2$), and another batch of the product as 90% pure E-isomer (0.18 g), mp 115-116°C, pmr (DMSO) δ: 7.71-7.68 (m, 5H, aromatic H), 6.80 (d, J=7.8 Hz, $H_4$ of 10% Z), 6.70 (d, J=7.8 Hz, $H_4$ of 90% E), 6.10 (t, CH= of 90% E), 5.81 (t, CH= of 10% Z), 2.26-2.40 (m, 8H, $CH_2$ and $3NCH_2$), 1.60 (m, 4H, $2CH_2$).

| | |
|---|---|
| Analysis: | Calculated for $C_{21}H_{21}Br_2NO$: C, 54.45; H, 34.57; N, 3.02, Br, 34.50. |
| Found (Z/E=98:2): | C, 54.51; H, 4.59; N, 3.01; Br, 34.45. |
| Found (Z/E=10:90): | C, 54.51; H, 4.57; N, 3.04; Br, 34.40. |

EXAMPLE 19

(Z)-1-[2-(6,11-Dihydrodibenz[b,e]-oxepin-11-ylidene)ethyl]pyrrolidine (Compound R)

A solution of n-butyl lithium in hexane (1.6 M, 3 mL) was added dropwise to a solution of 1.5 g pure (Z)-1-[2-(2-bromo-6,11-dihydrodibenz[b,e]-oxepin-11-ylidene) ethyl]pyrrolidine in 100 mL of dry THF at -70°C under a nitrogen atmosphere. After the yellowish orange solution was stirred at -70°C for ten minutes, gaseous carbon dioxide was bubbled through the reaction medium to give a pale yellow solution. The solution was allowed to warm gradually to room temperature and then concentraed under reduced pressure. None of the carbonylated products were able to be detected by HPLC on C18. The foamy residue was suspended in water, and the solids were collected by filtration. The solid obtained was recrystallized from water to give debrominated Z-isomer (0.08 g), m.p. 203-205°C. pmr ($CD_3OD$) δ: 7.30-7.40 (m, 4H, aromatic H); 7.24-7.27 (m, 1H, aromatic H); 6.90-7.07 (m, 3H, aromatic H); 5.84 (t, 1H, CH=); 5.25 (br s, 2H, $ArCH_2O$); 4.19 (d, J=6.5 Hz, 2H, $NCH_2C$=); 3.30 (m, 4H, $2NCH_2$); 2.04 (m, 4H, $2CH_2$).

| | |
|---|---|
| Analysis: | Calculated for $C_{20}H_{21}NO$ 1.25 $H_2O$: C, 68.56; H, 7.05; N, 4.00. |
| Found: | C, 68.62; H, 6.94; N, 3.98. |

EXAMPLE 20

1-((E)-3-(6-((E)-3-(1-Pyrrolidinyl)-1-(4-tolyl)-1-propenyl)-2-pyridyl) acryloyl) pyrrolidine.

Acrivastine (900mg, 2.58mmol) was dissolved in 20mL of dichloromethane under nitrogen and cooled in an ice bath. Thionyl chloride (0.34mL, 4.7mmol) was added and the reaction was allowed to stir for 30 minutes with cooling. Pyrrolidine (1.30mL, 15.5mmol) was added and the reaction was warmed to room temperature and stirred for 16 hours. The reaction solution was washed with three 20mL portions of 1.0 M aqueous sodium hydroxide, dried over sodium sulphate, and the solvent was removed under vacuum. The crude product was purified by chromatography on silica gel with 0-1% methanol in dichloromethane as eluent. The purified product was obtained as a dark oil which gave 170mg (16%) of beige crystals (m.p. 114.5-116°C) after two recrystallisations from acetonitrile. Calc. for $C_{26}H_{31}N_3O \cdot 0.02\ H_2O$; C, 77.08; H, 7.81; N, 10.37. Found: C, 77.14; H, 7.72; N, 10.44. NMR ($CDCl_3$), 200MHz, ppm downfield from TMS): 1.77(shrp mult,4H), 1.85-2.1(mult,4H), 2.40(s,3H), 2.53(shrp mult,4H), 3.21(d,2H,J=7), 3.55-3.75(mult,4H), 6.86-(d,1H,J=8), 7.05-7.25(mult,6H), 7.39 and 7.64 (ABq,2H,J=15), 7.49(t,1H,J=7.5).

EXAMPLE 21

(E)-Isopropyl 3-(6-(E)-3-(1-pyrrolidinyl-1-(4-tolyl)-1-propenyl)-2-pyridyl)acrylate

Acrivastine (1.01g, 2.90mmol) was dissolved in 50mL of isopropyl alcohol with 0.67g of conc. sulphuric acid and heated to reflux under nitrogen for 28 hours. The reaction was cooled to room temperature, diluted with 50mL of diethyl ether, washed with two 30mL portions of 1.0 M aqueous sodium hydroxide followed by saturated aqueous sodium chloride, and dried over sodium sulphate. The solvent was removed under vacuum and the residue was purified by chromatography on silica gel with 0-1% methanol in dichloromethane as eluent to give 0.57g of dark oil which crystallised upon cooling. The product was recrystallised from acetonitrile to give 220mg (19%) of beige crystals (m.p. 88-89°C). Calc. for $C_{25}H_{30}N_2O_2$: C, 76.89; H, 7.74; N, 7.17. Found: C,76.72; H, 7.81; N, 7.08. NMR ($CDCL_3$, 200MHz, ppm downfield from TMS): 1.32(d,6H,J=6.2), 1.78(shrp mult,4H), 2.40 (s,3H), 2.55(shrp mult,4H), 3.21(d,2H,J=7.0), 5.15-(septet,1H, J=6.2), 6.82(d,1H,J=7.2), 7.02(d,1H,J=15.6), 7.09(d,2H,J=8), 7.15-7.25(mult,4H), 7.50-(t,1H,J=7.8), 7.65(d,1H,J=15.6).

EXAMPLE 22

(E)-Butyl 3-(6-(E)-3-(1-pyrrolidinyl-1-(4-tolyl)-1-propenyl)-2-pyridyl) acrylate

Acrivastine (2.00g, 5.74mmol) was dissolved in n-butanol (200mL) with 0.63g of concentrated sulphuric acid and heated to reflux under nitrogen for 72 hours. The reaction was cooled to room temperature and neutralised with addition of saturated aqueous sodium bicarbonate. The solvent was removed under vacuum and the residue diluted with 50mL of diethyl ether, washed with 30mL of water followed by saturated aqueous sodium chloride, and dried over sodium sulphate. The solvent was removed under vacuum and the residue was purified by chromatography on silica gel with 0-1% methanol in dichloromethane as eluent to give a dark oil. The hydrochloride salt was prepared by dissolving the oil in ethanol and adding 0.2M ethanolic hydrogen chloride to pH=4.5. Addition of diethyl ether and chilling the solution gave 0.380g (15%) of light brown crystals (m.p. 119.5-121°C). Calc. for $C_{26}H_{32}N_2O_2$. HCl.0.25 $H_2O$: C, 70.10; H, 7.58; N, 6.29; Cl, 7.96. Found: C, 70.12; H, 7.60; N, 6.39; Cl, 7.89. NMR (amine form, $CDCl_3$, 200MHz, ppm downfield from TMS): 0.97(t,3H,J=7), 1.45(sextet,2H,J=7), 1.77(brs,s,(4H) over quintet (2H,J=7)), 2.40(s,3H), 2.52-(brd,s,4H), 3.19(d,2H,J=7), 4.22(t,2H,J=7), 6.82(d,1H,J=8), 7.04(d,1H,J=15.6), 7.08(d,2H,J=8), 7.15-7.25 (mult,4H), 7.50(t,1H,J=8), 7.66(d,1H,J=15.6).

EXAMPLE 23

In Vitro Cytotoxicity of Potentiating Agents in Chinese Hamster Ovary Cells

Chinese hamster ovary (CHO) tissue culture cells were obtained from Dr. Vic Ling, Princess Margaret Hospital, Toronto, Canada. The parental cell line (AuxB1) and a multidrug resistant line (C5S32) having an amplified form of the MDR drug transport protein were plated into 96-well microtitre culture dishes at 250 or 500 cells per well in minimal essential medium, type alpha, 10% fetal calf serum and incubated in 95%

oxygen/5% carbon dioxide for 48 hours. After this period, the medium was changed and one-half of the culture was treated with Actinomycin D (Act D) (0.01 M for AuxB1 cells and 0.5 M for C5S32 cells). C5S32 cells are about 200-fold resistant to Actinomycin D compared to the parental AuxB1 cell line. In addition to Act D some of the cultures also received a dose of the potentiating agent at 1 and 10 M. Thus, four conditions were tested in each screening assay: untreated cells in medium alone, cells receiving Act D alone, cells incubated with the potentiating agent alone, and cells incubated with a combination of Act D and the potentiating agent. Both the parental and mdr cell lines were treated with these four conditions simultaneously. Each experimental condition reported below is based on the average absorbance from eight replicate samples. The incubation with Act D and the test drug continued for 96 additional hours, after which 0.5 mg/ml MTT dye was added to the cultures and allowed to incubate for three hours. The cells were solubilized by addition of DMSO and the absorbance at 570 nm was monitored. The absorbance is directly related to the number of surviving cells in the culture dish.

In Table 1 below, the absorbance was normalized so that cytotoxicity of the potentiating agent could be evaluated. Untreated cultures were given a value of 1.00 and the cultures receiving 1 and 10 M of the potentiating agent are reported as a fraction of this value. To evaluate the compounds for inducing synergism with Actinomycin D, the absorbance values of cultures receiving Act D alone were assigned a value of 1.00 and cultures receiving the combination of Act D and potentiating agent Act D are reported as a fraction of this control. In most experiments, this concentration of Act D gave a reduction in cell number 10-20% below the value of completely untreated cultures.

TABLE 1

In Vitro Cytotoxicity of Potentiating Agents in Chinese Hamster Ovary Cells

| | | Relative Absorbance | | | |
|---|---|---|---|---|---|
| | | Wildtype AUX B1 | | Drug Resistant C5532 | |
| | | -ACT D | +ACT D | -ACT D | +ACT D |
| Untreated cell culture | | 1.0 | 1.0 | 1.0 | 1.0 |
| Test Compound | Dose | | | | |
| (A) | 1 M | 0.90 | 0.77 | 0.96 | 0.67 |
| | 10 M | 0.69 | 0.47 | 0.41 | 0.07 |
| (B) | 1 M | 1.00 | 1.00 | 1.00 | 1.00 |
| | 10 M | 1.00 | 0.86 | 0.86 | 0.49 |
| (C) | 1 M | 0.91 | 0.52 | 0.30 | 0.42 |
| | 10 M | 0.73 | 0.04 | 0.05 | 0.03 |
| (D) | 1 M | 0.78 | 0.61 | 1.00 | 0.93 |
| | 10 M | 0.79 | 0.50 | 1.00 | 0.66 |
| (E) | 1 M | 0.74 | 0.67 | 0.90 | 0.92 |
| | 10 M | 0.65 | 0.37 | 0.76 | 0.29 |
| (F) | 1 M | 0.93 | 0.82 | 0.61 | 0.71 |
| | 10 M | 0.96 | 0.52 | 0.15 | 0.15 |

| | | | | | |
|---|---|---|---|---|---|
| (G) | 1 M | 0.97 | 0.48 | 0.56 | 0.93 |
| | 10 M | 0.96 | 0.18 | 0.27 | 0.11 |
| (J) | 1 M | 0.92 | 0.63 | 0.77 | 0.63 |
| | 10 M | 0.93 | 0.29 | 0.52 | 0.14 |
| (L) | 1 M | 0.98 | 1.00 | 0.83 | 0.82 |
| | 10 M | 0.87 | 0.62 | 0.55 | 0.37 |
| (M) | 1 M | 1.00 | 0.92 | 1.00 | 0.90 |
| | 10 M | 0.92 | 0.95 | 1.00 | 0.54 |
| (N) | 1 M | 1.00 | 1.00 | 0.89 | 0.71 |
| | 10 M | 1.00 | 0.99 | 0.34 | 0.31 |
| (O) | 1 M | 1.00 | 1.00 | 0.85 | 0.66 |
| | 10 M | 1.00 | 0.98 | 0.48 | 0.35 |
| (P) | 1 M | 1.00 | 0.96 | 0.82 | 0.68 |
| | 10 M | 0.88 | 0.64 | 0.28 | 0.19 |
| (Q) | 1 M | 1.00 | 1.00 | 0.54 | 0.47 |
| | 10 M | 1.00 | 0.65 | 0.15 | 0.10 |
| (R) | 1 M | 1.00 | 1.00 | 0.82 | 0.95 |
| | 10 M | 0.94 | 0.92 | 0.53 | 0.51 |
| (S) | 1 M | 0.90 | 0.89 | 0.95 | 0.77 |
| | 10 M | 0.92 | 0.21 | 1.00 | 0.36 |
| (T) | 1 M | 1.00 | 0.90 | 0.80 | 0.66 |
| | 10 M | 1.00 | 0.83 | 0.29 | 0.12 |
| (U) | 1 M | 0.83 | 0.82 | 1.00 | 0.79 |
| | 10 M | 0.87 | 0.03 | 1.00 | 0.28 |

| | | | | | | |
|---|---|---|---|---|---|---|
| (V) | 1 | M | 0.83 | 0.88 | 0.92 | 0.85 |
| | 10 | M | 0.81 | 0.60 | 0.99 | 0.42 |
| (W) | 1 | M | 0.79 | 0.88 | 0.86 | 0.83 |
| | 10 | M | 0.84 | 0.47 | 1.00 | 0.35 |
| (X) | 1 | M | 0.86 | 0.85 | 1.00 | 0.85 |
| | 10 | M | 0.83 | 0.46 | 0.80 | 0.33 |
| (Y) | 1 | M | 1.00 | 1.00 | 0.48 | 0.58 |
| | 10 | M | 0.93 | 0.90 | 0.35 | 0.38 |
| (Z) | 1 | M | 1.00 | 1.00 | 0.61 | 0.70 |
| | 10 | M | 1.00 | 1.00 | 0.31 | 0.38 |
| (AA) | 1 | M | 0.97 | 1.00 | 0.53 | 0.69 |
| | 10 | M | 0.93 | 1.00 | 0.33 | 0.37 |
| (AB) | 1 | M | 0.99 | 1.00 | 0.50 | 0.52 |
| | 10 | M | 0.95 | 0.93 | 0.11 | 0.11 |
| (AC) | 1 | M | 0.91 | 0.99 | 1.00 | 0.94 |
| | 10 | M | 0.88 | 0.64 | 1.00 | 0.58 |
| (AD) | 1 | M | 1.00 | 0.95 | 1.00 | 1.00 |
| | 10 | M | 1.00 | 0.70 | 1.00 | 0.53 |
| (AE) | 1 | M | 0.84 | 0.78 | 0.94 | 0.93 |
| | 10 | M | 0.79 | 0.53 | 0.74 | 0.24 |
| (AF) | 1 | M | 0.86 | 0.85 | 0.91 | 0.87 |
| | 10 | M | 0.77 | 0.53 | 0.75 | 0.20 |
| (AG) | 1 | M | 1.00 | 0.85 | 0.98 | 0.86 |
| | 10 | M | 1.00 | 0.81 | 0.55 | 0.48 |

| | | | | | | |
|---|---|---|---|---|---|---|
| (AH) | 1 | M | 1.00 | 0.64 | 0.68 | 1.00 |
| | 10 | M | 0.41 | 0.05 | 0.28 | 0.13 |
| (AI) | 1 | M | 1.00 | 1.00 | 1.00 | 1.00 |
| | 10 | M | 1.00 | 1.00 | 0.91 | 0.56 |

EXAMPLE 24

In Vitro Cytotoxicity of Potentiating Agents in Human KB Epidermoid Carcinoma Cells

The procedure for assaying the cytotoxicity of potentiating agents with human KB epidermoid carcinoma cells is essentially the same as the assay procedure described above for use with Chinese hamster ovary cells. In brief, KB 3-1 (wt) and KB V-1 (mdr) cells are plated at 500 cells/well in 96-well culture plates in Dulbecco's modified eagle medium, supplemented with 10% fetal calf serum. After 48 hours of incubation at 37° C, the media is changed and cells are treated with actinomycin D at 0.1 nM (3-1) or 20 nM (V-1). The test potentiating agent is introduced to one-half the untreated cultures and one-half the Act D treated cultures at 1 and 10 M. After 96 hours of additional incubation at 37° C, 0.5 mg/ml MTT dye is added, the cells are incubated for three hours, after which the cells are dissolved in DMSO, and the absorbance is then read at 570 nm. The data is given in Table 2 below.

TABLE 2

In Vitro Cytotoxicity of Potentiating Agents in Human KB Epidermoid Carcinoma Cells

| | | Relative Absorbance | | | |
|---|---|---|---|---|---|
| | | Wildtype KB 3-1 | | Drug Resistant KB V-1 | |
| | | -ACT D | +ACT D | -ACT D | +ACT D |
| Untreated cell culture | | 1.0 | 1.0 | 1.0 | 1.0 |
| Test Compound | Dose | | | | |
| (A) | 1 M | 0.93 | 0.92 | 0.70 | 0.98 |
| | 10 M | 0.77 | 0.70 | 0.33 | 0.01 |
| (B) | 1 M | 1.00 | 1.00 | 0.86 | 0.99 |
| | 10 M | 0.92 | 0.89 | 0.59 | 0.11 |
| (C) | 1 M | 0.97 | 0.97 | 0.91 | 0.91 |
| | 10 M | 0.40 | 0.46 | 0.12 | 0.01 |
| (D) | 1 M | 0.84 | 0.97 | 0.83 | 0.95 |
| | 10 M | 0.78 | 0.88 | 0.80 | 0.41 |
| (E) | 1 M | 0.76 | 0.94 | 0.75 | 0.85 |
| | 10 M | 0.60 | 0.80 | 0.38 | 0.14 |
| (F) | 1 M | 0.98 | 0.97 | 0.84 | 1.00 |
| | 10 M | 0.76 | 0.80 | 0.47 | 0.16 |

| | | | | | | |
|---|---|---|---|---|---|---|
| (G) | 1 | M | 1.00 | 1.00 | 0.75 | 0.83 |
| | 10 | M | 0.84 | 0.68 | 0.54 | 0.01 |
| (J) | 1 | M | 0.58 | 0.55 | 0.36 | 0.48 |
| | 10 | M | 0.37 | 0.45 | 0.14 | 0.04 |
| (K) | 1 | M | 0.99 | 0.88 | 1.00 | 0.99 |
| | 10 | M | 0.98 | 1.00 | 0.75 | 0.21 |
| (L) | 1 | M | 1.00 | 1.00 | 1.00 | 1.00 |
| | 10 | M | 0.83 | 0.91 | 0.44 | 0.01 |
| (M) | 1 | M | 1.00 | 0.78 | 1.00 | 0.97 |
| | 10 | M | 1.00 | 0.75 | 0.34 | 0.36 |
| (N) | 1 | M | 0.88 | 0.86 | 0.75 | 0.78 |
| | 10 | M | 0.51 | 0.65 | 0.36 | 0.01 |
| (O) | 1 | M | 0.89 | 0.85 | 0.88 | 0.74 |
| | 10 | M | 0.89 | 0.77 | 0.45 | 0.01 |
| (P) | 1 | M | 0.84 | 0.86 | 0.80 | 0.77 |
| | 10 | M | 0.78 | 0.68 | 0.59 | 0.12 |
| (Q) | 1 | M | 0.87 | 0.80 | 0.86 | 0.91 |
| | 10 | M | 0.74 | 0.87 | 0.70 | 0.35 |
| (S) | 1 | M | 0.99 | 0.99 | 0.98 | 0.95 |
| | 10 | M | 0.30 | 0.40 | 0.34 | 0.04 |
| (T) | 1 | M | 0.89 | 0.85 | 0.88 | 0.74 |
| | 10 | M | 0.80 | 0.77 | 0.45 | 0.01 |
| (U) | 1 | M | 0.85 | 0.77 | 0.86 | 0.88 |
| | 10 | M | 1.00 | 0.03 | 0.08 | 0.13 |

| | | | | | | |
|---|---|---|---|---|---|---|
| (V) | 1 | M | 1.00 | 1.00 | 0.98 | 1.00 |
| | 10 | M | 0.97 | 0.94 | 0.51 | 0.23 |
| (W) | 1 | M | 1.00 | 1.00 | 0.95 | 1.00 |
| | 10 | M | 0.96 | 1.00 | 0.65 | 0.31 |
| (Y) | 1 | M | 0.79 | 0.81 | 0.74 | 0.78 |
| | 10 | M | 0.64 | 0.73 | 0.07 | 0.06 |
| (Z) | 1 | M | 0.65 | 0.72 | 0.73 | 0.72 |
| | 10 | M | 0.65 | 0.71 | 0.52 | 0.58 |
| (AA) | 1 | M | 0.92 | 0.91 | 0.86 | 0.83 |
| | 10 | M | 0.77 | 0.78 | 0.80 | 0.65 |
| (AB) | 1 | M | 0.78 | 0.84 | 0.87 | 0.90 |
| | 10 | M | 0.36 | 0.34 | 0.06 | 0.02 |
| (AC) | 1 | M | 0.77 | 0.79 | 0.89 | 0.84 |
| | 10 | M | 0.67 | 0.72 | 0.72 | 0.48 |
| (AD) | 1 | M | 0.86 | 0.90 | 0.96 | 0.92 |
| | 10 | M | 0.81 | 0.83 | 0.84 | 0.64 |
| (AH) | 1 | M | 1.00 | 0.96 | 0.70 | 0.85 |
| | 10 | M | 0.99 | 0.97 | 0.73 | 0.80 |
| (AI) | 1 | M | 0.97 | 0.89 | 0.77 | 0.85 |
| | 10 | M | 0.86 | 0.92 | 0.56 | 0.80 |

EXAMPLE 25

The following examples illustrate pharmaceutical formulations, wherein the active compound is a compound of formula (IVa) or (IVb) intended for treating allergic conditions. Compounds of formula (I) for use as antitumour potentiating agents may be formulated in similar manner, using appropriate dosages as described hereinbefore.

| Ingredient | Amount per ampoule |
|---|---|
| Active Compound<br>Water for Injections, q.s. | 1.0 mg<br>1.0 mL |

The finely ground active compound is dissolved in the water for Injections. The solution is filtered and sterilized by autoclaving.

| (B)-Syrup | |
|---|---|
| Ingredient | Amount per mL |
| Active Compound | 1.0 mg |
| Ethanol | 0.3 mg |
| Sucrose | 2.0 mg |
| Methylparaben | 0.5 mg |
| Sodium Benzoate | 0.5 mg |
| Cherry Flavour | q.s. |
| Colouring | q.s. |
| Water | Q.S. to 5.0 mL |

Ethanol, sucrose, sodium benzoate, methylparaben, and flavouring are combined in 70% of the total batch quantity of water. Colouring and the active compound are dissolved in the remaining water, then the two solutions are mixed and clarified by filtration.

| (C)-Tablet | |
|---|---|
| Ingredient | Amount per Tablet |
| Active Compound | 1.0 mg |
| Lactose | 110.0 mg |
| Corn Starch, Pregelantinized | 2.5 mg |
| Potato Starch | 12.0 mg |
| Magnesium stearate | 0.5 mg |

The active compound is finely ground and intimately mixed with the powered excipients lactose, corn starch, potato starch and magnesium stearate. The formulation is then compressed to afford a tablet weighing 126 mg.

| (D)-Capsule | |
|---|---|
| Ingredient | Amount per Capsule |
| Active Compound | 1.0 mg |
| Lactose | 440.0 mg |
| Magnesium Stearate | 5.0 mg |

The finely ground active compound is mixed with the powdered excipients lactose and magnesium stearate and packed into gelatin capsules.

| (E)-Nasal Spray | |
|---|---|
| Ingredient | Amount per 100.0 mL |
| Active Compound | 1 g |
| Sodium Chloride | 0.8 g |
| Preservative | 0.5 g |
| Purified Water q.s. | 100.0 mL |

The preservative is dissolved in warm purified water and after cooling to 25-30°C the sodium chloride and active compound are added. The pH is then adjusted to 5.5-6.5 and purified water is added to bring the final volume to 100.0 mL.

**Claims**

**1.** A compound of formula (IVa)

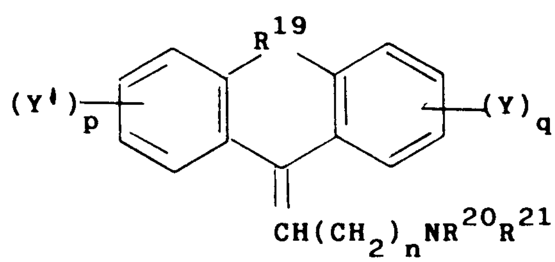

(IVa)

wherein:

$R^{19}$ is $-CH_2CH_2-$, $-CH_2O-$, $-OCH_2-$, $-NHCH_2-$, or $-CH_2NH-$;
Y is hydrogen or halogen attached at the 1, 2, 3 or 4 position;
Y' is hydrogen or halogen attached at the 7, 8, 9 or 10 position;
n is 0 to 3;
each of p and q is 1 to 4; and
$R^{20}$ and $R^{21}$ independently are hydrogen, or $C_{1-4}$ alkyl, or together with the nitrogen atom form a nitrogen-containing heterocyclic ring having four to six ring members, with the provisos that
a) when $R^{19}$ is $-CH_2CH_2-$, $-CH_2O-$ or $-OCH_2-$, p and q are each 1 and one of Y and Y' is halogen, the other is not hydrogen; and
b) when $R^{19}$ is $-CH_2CH_2-$ or $-CH_2O-$, p and q are both 1 and each of Y and Y' is hydrogen or halogen, then n is not 2;
or a pharmaceutically acceptable salt thereof.

**2.** A process for the preparation of a compound of formula (IVa) as defined in claim 15 which comprises:
a) reaction of a compound of formula (VIII):

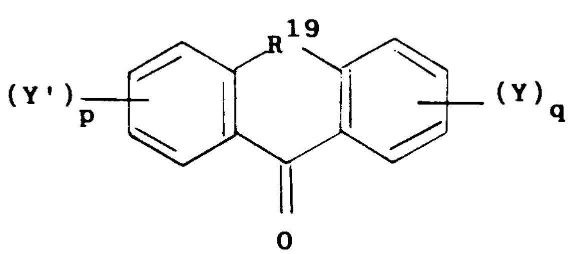

(VIII)

with an appropriate Wittig reagent, or with an appropriate Grignard reagent followed by dehydration;
b) halogenation of a compound of formula (IX);
c) conversion of one compound of formula (IV) into a different compound of formula (IV) by halogen

exchange.

**3.** A compound of formula (IVb):

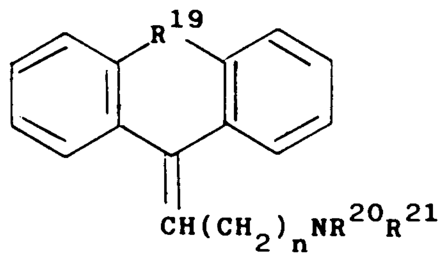

(IVb)

wherein:

$R^{19}$ is $-CH_2CH_2-$, $-CH_2O-$, $-OCH_2-$, $-NHCH_2-$, or $-CH_2NH-$;
Y is hydrogen or halogen attached at the 1, 2, 3 or 4 position;
Y' is hydrogen or halogen attached at the 7, 8, 9 or 10 position;
n is 0 to 3;
each of p and q is 1 to 4; and
$R^{20}$ and $R^{21}$ independently are hydrogen, or $C_{1-4}$ alkyl, or together with the nitrogen atom form a nitrogen-containing heterocyclic ring having four to six ring members,

with the proviso that when $R^{19}$ is $-CH_2CH_2-$ or $-CH_2O-$, p and q are both 1 and each of Y and Y' is hydrogen or halogen, then n is not 2,

or a pharmaceutically acceptable salt thereof.

for use as a therapeutic agent.

**4.** A pharmaceutical formulation comprising a compound of formula (IVa) or (IVb) or a pharmaceutically acceptable salt thereof together with a pharmaceutically acceptable carrier therefor.